# EUROPEAN PATENT SPECIFICATION

(11) **EP 3 264 298 B1**
(45) Date of publication and mention of the grant of the patent: **08.04.2020**
(21) Application number: 16176701.7
(22) Date of filing: 28.06.2016
(51) Int. Cl.: G16H 20/40

(54) **RADIOTHERAPY INFORMATION SYSTEM WITH TREATMENT PLAN EVALUATION**
STRAHLENTHERAPIEINFORMATIONSSYSTEM MIT BEHANDLUNGSPLANBEWERTUNG
SYSTÈME D'INFORMATION DE RADIOTHÉRAPIE AVEC ÉVALUATION DE TRAITEMENT DE PLAN

(43) Date of publication of application: 03.01.2018
(73) Proprietor: Ebit srl, 16152 Genova (IT)
(72) Inventor: Minetti, Irene, 15074 Molare (AL) (IT); Ferrando, Giovanni, 16156 Genova Pegli (IT)
(74) Representative: Bessi, Lorenzo

(56) References cited:
- EP-A1- 2 078 537
- WO-A2-2006/130863
- US-A1- 2011 107 270
- US-A1- 2016 113 608

## Description

### BACKGROUND OF THE INVENTION

Embodiments herein generally relate to radiotherapy information systems, and more particularly to radiotherapy information systems integrated with picture archive communications systems (PACS) for evaluation and review of radiotherapy treatment plans.

Radiation Therapy Work Flow. Radiation therapy generally comprises three modalities: external beam therapy, nuclear medicine, and brachytherapy. Upon being referred by a general physician for consultation, the patient is scheduled for an appointment. On the appointed day, the patient registers at the oncology department. The radiation oncologist sees the patient and determines the most appropriate modality for the patient on the basis of prior investigation results and treatment protocol. In general, radiation therapy entails treatment planning and delivery. Thus, when the oncologist decides that the patient should proceed with radiation therapy, the patient will be scheduled for treatment planning before undergoing treatment. External beam therapy accounts for over 90% of the workload in a radiation therapy department. Brachytherapy often plays a supplementary role and is often used in the treatment of gynecologic cancers. Nuclear medicine has become independent of radiation therapy in many hospitals.

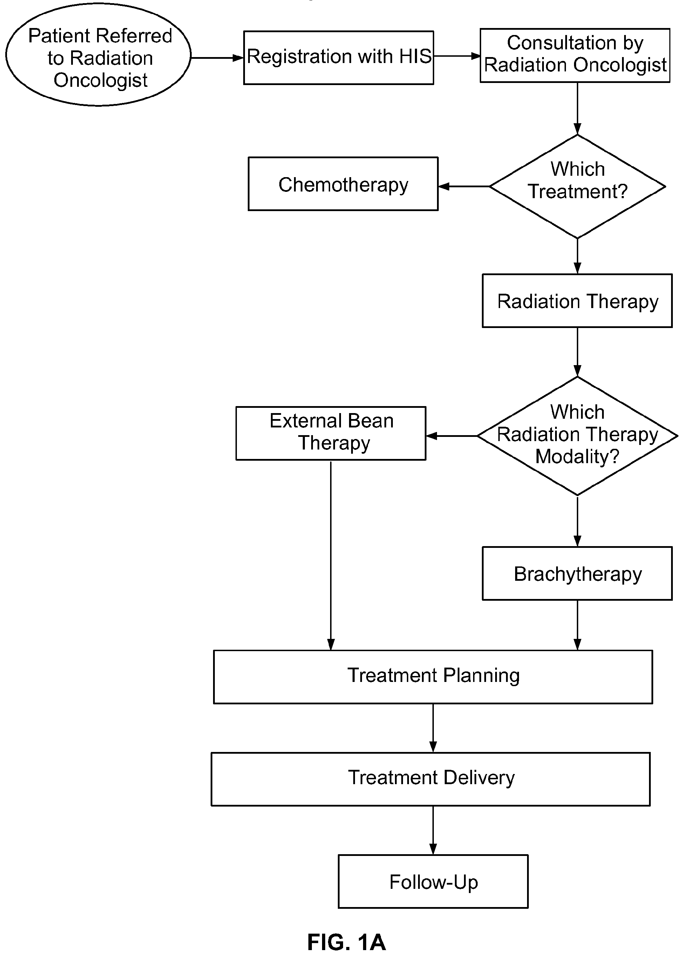

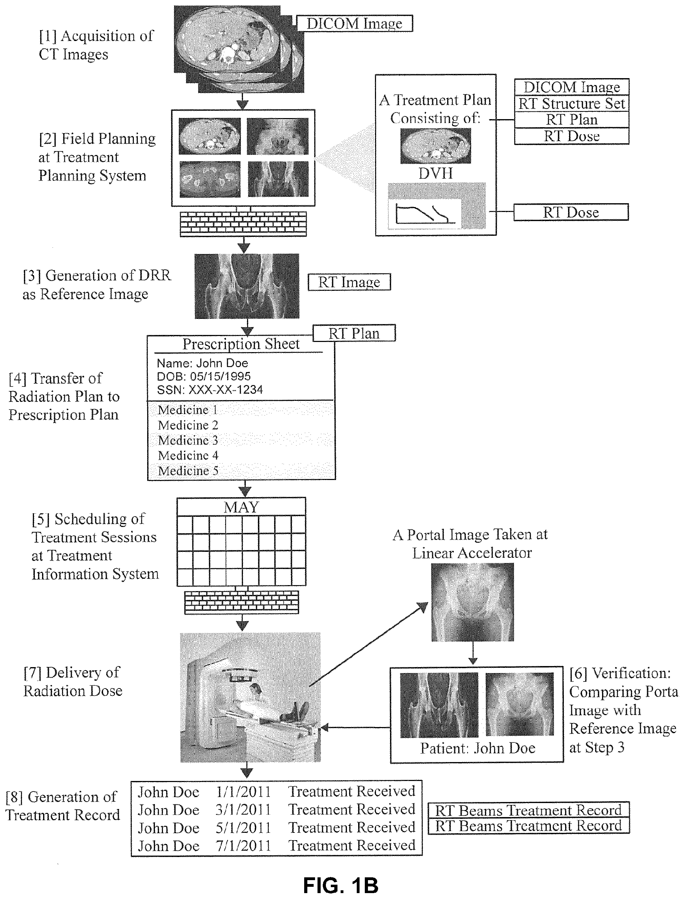

FIG. 1A provides a brief overview of patient treatment in a radiation oncology department once the type of treatment has been determined. In some cases, the patient may receive both chemotherapy and radiation therapy. FIG. 1B shows, as an example, the general procedures involved in the planning and delivery of external beam therapy in a specific clinical case of prostate cancer.

Treatment Planning (Steps 1-5).- A goal of treatment planning is to deliver a desired uniform radiation dose to a tumor-bearing site, while limiting or avoiding delivery of dose to surrounding healthy tissue, and in particular radiosensitive structures (e.g., the urinary bladder and rectum in cases of prostate cancer). An example of a treatment planning system is disclosed in US 2011/107270.

Information from medical images is very helpful to the radiation therapy planning process. The process starts with the localization of the tumor volume. For this purpose, the oncologist will order a CT or MR study of the affected anatomic area. When using a CT study, the patient's information is delivered to the CT simulator room, where the radiation therapist positions the patient for scanning. The CT scans are generated and saved as DICOM images (image objects) and stored in a PACS or other database (step 1).

The CT scans are then transferred to a computer treatment planning system (TPS) (step 2) for radiation field planning. Previous diagnostic images, CT scans, magnetic resonance (MR) images, or positron emission tomographic (PET) scans may also be retrieved to aid in the delineation of tumor volume. At the TPS workstation, the tumor volume and the organs at risk (OARs) are delineated. Treatment fields of appropriate size and optimal gantry or collimator angles are determined. The TPS computes a radiation dose distribution within the body region to be treated (step 2). Determining the best radiation therapy plan requires a clinical judgment based on the balance between adequate target volume coverage and sparing of OARs. Each plan is evaluated carefully with the use of dose-volume histograms and planar dose distributions. Although, dose-volume histograms do not provide spatial information, dose-volume histograms provide a global view as to whether the resultant plan meets the dose-volume criteria.

A detailed slice-by-slice analysis of isodose distribution is utilized for examining target volume coverage and identifying the exact location of "hot" and "cold" spots with respect to radiation dose. When the OARs overlap with, or are in proximity to, the target volume, the dose is often limited by ranking the OARs in terms of relative importance. Images similar to projection images can be re-constructed from the CT scans to show the treatment field positions and are called digitally reconstructed radiographs (DRRs) (step 3). A DRR, or simulator image obtained for treatment planning, serves as a reference image for treatment verification later. The finished plan is presented to the radiation oncologist for evaluation and approval. If the plan is found satisfactory, the oncologist prescribes the treatment (step 4) on the radiation therapy prescription sheet. A treatment record with all treatment details is prepared with the prescription. Treatment sessions are scheduled in the treatment information system (step 5) and transferred to the radiation treatment unit or linear accelerator, either through the department network or by manual paper delivery.

Treatment Delivery (Steps 6-12).-Before the actual radiation treatment commences, the accuracy of the treatment plan in terms of field sizes, setup, shielding positions, and so on is verified at the linear accelerator. For such verification, a portal image is obtained at the linear accelerator (step 6), either as a film image or (with an electronic portal imaging device installed in the linear accelerator) as a digital portal image. The image is then compared with the reference DRR or simulator image. When the irradiated portal aligns correctly with the portal on the reference images, the oncologist approves the verification. Radiation treatment can then proceed (step 7). Otherwise, a repeat portal image may be requested. As one example, the patient may be treated five times a week for 7-8 weeks. At each treatment session, each of the treatment beams is recorded, as well as the radiation dose and the cumulative dose to date (step 8). During the course of treatment, weekly verification images are acquired to ensure accuracy. The oncologist also reviews the patient's progress and prescribes whatever medicine is required. Upon completion, the patient's course of treatment is summarized and filed. A follow-up appointment is usually made with the patient for review purposes. An example of a treatment delivery system is disclosed in EP2078537A1.

PACS generally provide image viewing and image processing stations (e.g., workstations) that are connected to one another via an image communication network. PACS receive and archive DICOM images from different types of diagnostic modalities and are often connected to specialist information systems, such as Radiology Information Systems (RIS), Cardiology Information Systems (CIS), used in departments that produce medical images. In such cases, when clinicians analyze the clinical information to make a diagnosis and to provide the therapeutic indications, the integration between the management system and the image viewer guarantees a complete and ergonomic workstation guiding the physicians through the whole reporting workflow. Radiology and cardiology mainly involves the generation of medical images and reporting of the diagnosis, whereas in radiation therapy, the images are used for treatment planning and specific kinds of images are generated for treatment verification. In radiology, the procedure is considered complete as soon as the imaging findings have been reported, but this is only the beginning for radiation therapy. PACS are used to store various types of DICOM images (CT, DX, CX, X-ray, ultrasound, MR and other studies), but heretofore PACS have not been integrated with the RT EPR systems.

Also, the Digital Imaging and Communications in Medicine (DICOM) standard has been extended for use in various sub-specialties. One extension was applied to radiation therapy and is known as DICOM-RT. In addition to the protocol used in the DICOM standard, seven DICOM-RT objects-namely, RT Image, RT Structure Set, RT Plan, RT Dose, RT Beams Treatment Record, RT Brachy Treatment Record, and RT Treatment Summary Record-have been created, each with a well-defined data model. The data models set the standard for integration of radiation therapy information for an electronic patient record and facilitate the interoperability of different radiation therapy systems, thus making possible the sharing of information from different systems. The DICOM RT objects can be exported from TPS and stored in PACS.

However, existing solutions experience certain limitations. First, conventional systems offer limited electronic patient folder (EPR) capabilities related to radiotherapy management workflow. In addition there is no integration between the various types of systems involved in radiotherapy workflow-such as Radiotherapy EPR, RT PACS, Treatment Planning Systems (TPS), Record & Verify Systems. Accordingly, the physician's experience is limited when he/she wants to review the complete clinical history of a patient at one point in time, for example during follow-up check-ups. In fact, a treatment planning system usually does not save, nor export, all information that are displayed while generating a particular treatment plan. For example, a treatment planning system may not export dose volume histograms (DVH) that were visualized and used by a physician or other healthcare provider when defining the treatment plan. Consequently, when a treatment plan workflow is reviewed in a radiotherapy EPR system, even when this is integrated with PACS RT viewer, it may not be possible to correlate all the information necessary to evaluate the clinical evolution of the patient status.

Even when isodoses lines are exported within RT DOSE Objects, the isodose lines are saved with a space resolution different from the CT / MR space resolution. Therefore, in general, conventional PACS viewers are unable to correctly overlay the isodoses lines on CT slices. Therefore, a need remains for improved systems and methods that address the above noted difficulties, as well as other problems that will become available upon reading the text herein.

### BRIEF DESCRIPTION

In accordance with embodiments herein, a method for providing a more complete radiotherapy workflow management is provided. Starting from the following DICOM objects-exported by TPS:
RT Dose IOD (see DICOM PS3.3 2015a-A.18): Distribution of doses calculated by the TPS. According to the standard, they can be represented by grids, isodose curves or points. Moreover, according to the standard, the object may contain the DVH but this is not a required module.
RT Structure Set IOD (see DICOM PS3.3 2015a-A.19). Patient anatomical structures defined as contours on the reference series.
RT Plan IOD (see DICOM PS3.3 2015a-A.20). Definition of the treatment plan. In the treatment it is possible to define some fractions to divide the treatment.

The method obtains a reconstruction of isodoses curves to be displayed in the PACS viewer overlayed to the reference study (CT or MR). The isodose curves represent contours of regions with a dose greater than a select value. The reconstruction regards several slices of the reference volume and allows to assign to each voxel of the volume the related dose value. Further, the method calculates dose-volume histogram (DVH) and store it into the PACS, as DICOM RT object.

Optionally, the method may further comprise performing a 3-D reconstruction to calculate isodose curves that are visualized not only on the axial plane, but also on coronal and sagittal planes. Optionally, the system is natively integrated with a PACS system or a Healthcare Information System dedicated to the workflow management in a radiotherapy department.

In accordance with embodiments herein a method is provided for providing radiotherapy workflow management. The method obtains an image object that includes a volumetric data set of voxels having a spacing and position relative to an image coordinate system. The method obtains RT objects that include anatomical information regarding anatomical structures of treated regions and including dose data defining dose values for dose points in the treatment region. The dose points are misaligned with the voxels in the volumetric data set. The method calculates a transformation to align and/or interpolate the dose points with voxels in the volumetric data set and calculates at least one of dose-volumetric histogram (DVH) data or isodose curves based on the volumetric data set and the dose values. The method displays, through a workstation viewer, image slices from the volumetric data set with dose indicia superimposed on the image slices. The dose indicia is indicative of the dose data and is based on the at least one of DVH data or the isodose curve.

Optionally, the method further comprises presenting the dose indicia for treatment regions having dose values greater than a select dose value (e.g. isodose curves). The dose points may represent plan dose points in a treatment plan. The plan dose points and voxels may have different first and second resolutions, the transformation aligning the plan dose points and voxels. The dose points may represent delivery dose points from a radiotherapy delivery system. The delivery dose points and voxels may have different first and second resolutions, the transformation aligning the delivery dose points and voxels. Optionally, the transformation may include performing interpolation between dose points to align the dose points with the voxels. The transformation may include changing a target dose resolution of the dose points to align with an image voxel resolution of the volumetric data set.

In at least some embodiments, a first set of RT objects include the anatomical information and a second set of the RT objects include the dose data. The RT objects may include a set of DICOM RT objects generated by a therapy planning system. The set of DICOM RT objects may represent the external system. The set of DICOM RT objects may include information about the anatomical structure of the treatment region defined relative to the image coordinate system of the volumetric data set. The set of DICOM RT objects may represent information about the planned doses for a plurality of points distributed throughout the treatment region, where at least a portion of the points are misaligned with the voxels in the volumetric data set. The displaying may include displaying the image slices along axial, coronal and sagittal views planes, with the dose indicial overlaid thereon. The RT objects may be generated by one or more external planning systems, the obtaining operation including accessing a DICOM database that stores the RT objects.

In accordance with embodiments herein, a system for providing radiotherapy workflow management is provided. The system comprises a workstation and a communications interface configured to obtain an image object that includes a volumetric data set of voxels having a spacing and position relative to an image coordinate system. The communications interface is configured to obtain RT objects that include anatomical information regarding an anatomical structure of a treatment region and include dose data defining dose values for dose points in the treatment region, the dose points misaligned with the voxels in the volumetric data set. The system further comprises memory storing program instructions and a processor configured to execute the program instructions to: calculate a transformation to align the dose points with voxels in the volumetric data set; calculate at least one of dose-volumetric histogram (DVH) data or an isodose curve based on the volumetric data set and the dose values; and direct the workstation to display image slices from the volumetric data set with dose indicia superimposed on the image slices, the dose indicia indicative of the dose data and is based on the at least one of DVH data or the isodose curve.

Optionally, the processing may be further configured to present the dose indicia for treatment regions having dose values greater than a select dose value. For example, isodose curve may be illustrated for dose values that equal or exceed a select dose value, such as by overlaying the dose indicia on one or more images associated with CT slices.

The dose points may represent plan dose points in a treatment plan. The plan dose points and voxels may have different first and second resolutions, the transformation aligning the plan dose points and voxels. The dose points may represent delivery dose points from a radiotherapy delivery system. The delivery dose points and voxels may have different first and second resolutions, the transformation aligning the delivery dose points and voxels. The transformation may include changing a target dose resolution of the dose points to align with an image voxel resolution of the volumetric data set. Additionally or alternatively, the transformation may include performing interpolation between dose points (thereby forming interpolated dose points) to form the target dose resolution to align with an image voxel resolution of the volumetric data set.

Optionally, a first set of RT objects may include the anatomical information and a second set of the RT objects may include the dose data. The RT objects may include a set of DICOM RT objects generated by a therapy planning system. The set of DICOM RT objects may represent the external system. The set of DICOM RT objects may include information about the anatomical structure of the treatment region defined relative to the image coordinate system of the volumetric data set. The RT objects may include a set of DICOM RT objects generated by a therapy planning system. The set of DICOM RT objects may represent information about the planned doses for a plurality of points distributed throughout the treatment region, where at least a portion of the points are misaligned with the voxels in the volumetric data set. The displaying may include displaying the image slices along axial, coronal and sagittal views planes, with the dose indicial overlaid thereon. The system further comprising a DICOM database that may store the RT objects and the image objects.

Optionally, the system provides the access to DICOM images (volumetric data set and dose information) directly from the RT EPR. While checking the anamnestic patient information, the clinical status and the planned treatment, the radiotherapist/oncologist can open contextually with a single click the image-based information and access to the representations and the data described in the previous points.

### BRIEF DESCRIPTION OF THE DRAWINGS

FIG. 1A provides a brief overview of the work flow in an oncology - radiotherapy - department from the patient arrival and along the care process in accordance with embodiments herein.
FIG.1B shows, as an example, the general procedures involved in the planning and delivery of external beam therapy in a specific clinical case of prostate cancer in accordance with embodiments herein.
FIG. 2A illustrates a block diagram of a healthcare environment in accordance with embodiments herein.
FIG. 2B illustrates an example block diagram of the RT-EPR subsystem in accordance with embodiments herein.
FIG. 3 illustrates an example overall radiotherapy workflow implemented in accordance with an embodiment herein.
FIG. 4A illustrates a process carried out in accordance with embodiments herein to enable verification and accurate review of treatment plans and resulting therapy delivery data in accordance with embodiments herein.
FIG. 4B illustrates a process carried out in accordance with embodiments herein to enable verification and accurate review of treatment plans and resulting therapy delivery data in accordance with embodiments herein.
FIG. 5 illustrates an example of objects that may be operated upon in connection with the process of FIG. 4 in accordance with embodiments herein.
FIG. 6 illustrates an example candidate volume-dose overlay that may be analyzed in accordance with embodiments herein.
FIG. 7A illustrates differential DVH data for a four field prostate treatment plan for a target volume (a) and the rectum (b) in accordance with embodiments herein.
FIG. 7B illustrates a CT scan with superimposed dose indicia (color-coded) for radiation dose data generated in connection with therapy planning in accordance with an embodiment herein.
FIG. 7C illustrates a dose volume histogram corresponding to the information presented in FIG. 7B in accordance with an embodiment herein.
FIG. 8 illustrates isodose curves that may be calculated in accordance with embodiments herein.
FIG. 9 illustrates one example of a TPS, radiotherapy delivery system and imaging system utilized in accordance with embodiments herein.
FIG. 10A illustrates screenshots of the manner in which image slices are presented to a user in accordance with embodiments herein.
FIG. 10B illustrates screenshots of the manner in which image slices and dose indicia may be presented to a user in accordance with embodiments herein.
FIG. 10C illustrates screenshots of the manner in which image slices and dose indicia may be presented to a user in accordance with embodiments herein.
FIG. 10D illustrates screenshots of the manner in which image slices and RT structure outlines may be presented to a user in accordance with embodiments herein.

### DETAILED DESCRIPTION

The embodiments of the systems and methods described herein may be implemented in hardware or software, or a combination of both. However, preferably, these embodiments are implemented in computer programs executing on programmable computers each comprising at least one processor, a data storage system (including volatile and non-volatile memory and/or storage elements), at least one input device, and at least one output device. For example and without limitation, the programmable computers may be a personal computer, laptop, personal data assistant, and cellular telephone. Program code is applied to input data to perform the functions described herein and generate output information. The output information is applied to one or more output devices, in known fashion.

Each program is preferably implemented in a high level procedural or object oriented programming and/or scripting language to communicate with a computer system. However, the programs can be implemented in assembly or machine language, if desired. In any case, the language may be a compiled or interpreted language. Each such computer program is preferably stored on a storage media or a device readable by a general or special purpose programmable computer, for configuring and operating the computer when the storage media or device is read by the computer to perform the procedures described herein. The inventive system may also be considered to be implemented as a computer-readable storage medium, configured with a computer program, where the storage medium so configured causes a computer to operate in a specific and predefined manner to perform the functions described herein.

### Acronyms and Abbreviations

The following (as well as additional) acronyms are used throughout:
DVH: Dose-Volume Histograms. A dose-volume histogram is a histogram relating radiation dose to tissue or anatomical structure volume in radiation therapy planning. DVH summarizes 3D dose distributions in a graphical 2D format. DVH can be visualized as differential or cumulative histograms.
TPS: Treatment Planning System. The systems used for treatment planning, which is then exported in DICOM file format by the system.
CTV: Clinical Target Volumes. A volume identified by 2D contours drawn on the slices of the reference CT, which will define the area to be treated on the basis of clinical considerations; this area includes the tumor mass and possible infiltrations.
DICOM: Digital Imaging and Communication in Medicine ("DICOM") standard as maintained by the National Electronic Manufacturer's Association ("NEMA").
PTV: Planned Target Volumes. A volume identified by 2D contours drawn on the reference CT which defines the area that will be radiated in order to treat the CTVs with the required doses.
PACS: Picture Archive Communications System.
EPR: Electronic Patient Record

### DETAILED DESCRIPTION

FIG. 1 illustrates a block diagram of a radiotherapy department. The environment includes one or more imaging systems 110, one or more treatment planning systems (TPS) 150, one or more radiotherapy delivery systems (RDS) 160, and one or more workstations 125. A radiotherapy electronic patient record (RT-EPR) system is integrated with a picture archive computing system (PACS), together collectively referred to as an RT-EPR-PACS 120. The RT-EPR-PACS 120 integrates data and images from numerous imaging systems 110 modalities (x-ray, CT, NM, MRI, PET, SPECT, ultrasound, etc.). The RT-EPR-PACS 120 is configured to receive and process images from various types of imaging systems, treatment planning systems, and the like. The RT-EPR-PACS is configured to receive images and other data from multiple healthcare providers, from different departments within a healthcare provider, etc. The RIS functionality within the RT-EPR-PACS accesses, stores, updates and manages electronic patient records, and includes one or more review modules for advanced image visualization and reporting. The PACS functionality within the RT-EPR-PACS provides long-term archive and distribution of diagnostic images and patient related data.

Image data 105 is acquired by one or more imaging systems 110 and stored in an image database 117 on an image server 115. Image data set 105 is then transferred to a DICOM database 113, using a communication protocol, such as one of the DICOM communication protocols. Optionally, the image data sets may be conveyed directly from the imaging system 110 to the DICOM database 113. The image data sets may represent two dimensional images and/or three dimensional volumetric data sets.

For each originally captured image data set, there may be one or more associated image data files. For example, digital mammography images can be digitally processed such that, when displayed, the key features required for mammographic interpretation will be demonstrated. Separate image data files will be used to store the original raw data and processed/reconstructed image data sets. The DICOM Digital Mammography X-Ray Image Information (MG) object, for example, includes one of two types of image data: "For processing" image data which has not been processed for interpretation; or "For presentation" image data which has been processed and is ready to be displayed. Processed image data files such as these will normally include a reference identifying the image data file of the original image.

Other data files stored in the DICOM database 113 may include information, referred to as non-image objects, relating to the way in which an image is to be presented. The non-image objects do not include image data but instead include other information related to the image data file(s) to which the non-image objects are to be applied. For example, the DICOM grayscale presentation state (GSPS) object stores the viewing parameters for a DICOM image objects 161 in order to allow for the consistent presentation of the images. A GSPS object may reference more than one image object 161 and, conversely, there may be more than one GSPS object which references a single image object. The DICOM database 113 also stores treatment plan objects 162, therapy delivery objects 164, structured reporting objects 166, as well as other forms of objects and data defined in accordance with the DICOM standard.

The structured reporting (SR) class of DICOM objects are used for transmission and storage of clinical documents. SR objects provide the capability to link text and other data to particular images. Computer-aided detection (CAD) SR objects, for example, include a reference to the image on which the detection was performed and allow the location of CAD findings to be displayed on the image. The data files stored in the DICOM database 113 are often saved into groups, called studies. Each study includes the data files which relate to one particular patient for a particular purpose. One study may include many different types of data files, including image data files such as DICOM Computed Tomography (CT) and Magnetic Resonance Imaging (MR) objects as well as non-image data files such as DICOM GSPS and SR objects.

The treatment planning system 150 is configured to enable one or more of radiation oncologists, radiation therapists, medical physicists and/or medical dosimetrists to plan an appropriate therapy. For example, the therapy may involve external beam radiotherapy, internal brachytherapy treatment techniques or other forms of treatment for patients with cancer, tumors, etc. In generally, the treatment planning system 150 utilizes one or more medical image set for treatment planning. The medical image set may be computed tomography images, magnetic resonance imaging images, positron emission tomography images and the like. The image set is used to form a virtual patient for a computer-aided design procedure. Treatment simulations are used to plan the geometric, radiological, and dosimetric aspects of the therapy using radiation transport simulations and optimization. For intensity modulated radiation therapy (IMRT), this process involves selecting the appropriate beam energy (photons, and perhaps protons), energy (e.g. 6 MV, 18 MV) and arrangements. For brachytherapy, involves selecting the appropriate catheter positions and source dwell times (in HDR brachytherapy) or seeds positions (in LDR brachytherapy). The more formal optimization process is typically referred to as forward planning and inverse planning. Plans are often assessed with the aid of dose-volume histograms, allowing the clinician to evaluate the uniformity of the dose to the diseased tissue (tumor) and sparing of healthy structures.

Once a treatment plan is developed, the treatment plan 152 may be saved to a treatment plan database 154 on a server 156. Additionally or alternatively, the treatment plan 152 may be saved in memory on the treatment planning system 150. The treatment plans 152 are conveyed to a DICOM database and saved as treatment plan objects 162. The DICOM database storing the treatment plan objects may represent the same or a different DICOM database as used to store the image objects. Additionally or alternatively, the treatment plans may be conveyed directly from the treatment planning system 150 to a DICOM database. The treatment plan objects may store various types of information as discussed hereafter in accordance with the DICOM standard.

The radiotherapy delivery system 160 delivers radiation therapy based on the treatment plan 152. Following delivery of the radiation therapy, therapy delivery data 158 may be saved with the therapy plan 152 in the database 154. Additionally or alternatively, the therapy delivery data 158 may be stored locally in memory at the radiotherapy delivery system 160. The therapy delivery data 158 may be transferred to a DICOM database and saved as a therapy delivery objects 164. Optionally, the therapy delivery data 158 may be transferred directly from the radiotherapy delivery system 160 to the DICOM database and saved as therapy delivery objects 164. Examples of treatment planning systems and radiotherapy delivery systems are described below in more detail.

It should be understood that the treatment planning system 150, radiotherapy delivery system 160 and RT-EPR-PACS system 120 may be implemented in hardware or software or a combination of both. With respect to the RT-EPR-PACS 120, modules of the radiotherapy information sub-system 122 are preferably implemented in computer programs executing on programmable computers each comprising at least one processor, a data storage system, and at least one input and at least one output device. Without limitation, the programmable computers may be a server, personal computer, laptop, personal data assistant or cellular telephone. In some embodiments, the radiotherapy information sub-system 120 is installed on the hard drive of a user workstation 125, such that the user workstation 125 operates with the RT-EPR-PACS system 120 in a client-server configuration. The RT-EPR-PACS system 120 and the user workstation 125 both include client and server aspects relative to different functions. (). By general, the RT-EPR-PACS system 120 is provided as a central server. Optionally, the structure and function of the RT-EPR-PACS system 120 may be implemented on a stand-alone workstation. In other embodiments, the RT sub-system 122 can run from a single dedicated workstation that may be associated directly with a particular imaging system 110. In yet other embodiments, the RT-subsystem 122 can be configured to run remotely on the user workstation 125 while communication with the PACS system occurs via a wide area network (WAN), such as through the internet.

The RT-EPR-PACS 120 is configured to access the image objects 161 stored in the DICOM database 113 in response to user commands through workstations 125, which may be a computer workstation consisting of a processor, display device and input devices (e.g., keyboard, mouse). The DICOM database 113 may represent a core component in many PACS systems. Alternatively, the workstation 125 may be a mobile device or any other suitable access device for displaying image data. Each image object 161 generated from the image data 105 can be expressed in logical parts. One part is known as pixel data that represents the displayed image. The other logical part is the metadata that represents a set of attributes that describes the image such as patient information, study grouping, and image attributes.

The RT-EPR-PACS 120 provides a radiotherapy workflow, in which various aspects of the workflow are presented through corresponding windows. In accordance with embodiments herein, the RT-ERS subsystem 122 includes an RT treatment plan (RTTP) visualization module 126 which presents the user with an RT treatment option plan. For example, the user may select the RT treatment plan by choosing an option/tab on a higher level workflow management window. When the option/tab is selected, the system launches the RTTP module 126 which is configured to provide a medical radiation therapist with an accurate tool that affords a clear description of one or more treatment plans that has been previously been defined utilizing one of the treatment planning systems 150.

The RTTP module 126 directly accesses a selected treatment plan object 162 (previously defined and stored) from the DICOM database 113. The RTTP module 126 also directly accesses one or more image objects 161 associated with the patient for whom the treatment plan has been developed. The RTTP module 126 implements processes described herein to afford a therapist with tools to effectively evaluate treatment plan object 162. As explained herein, the RTTP module 126 provides direct access to, or calculation of, dose volume histograms (DVH) and planned dose information. The DVH allow the therapist to evaluate easily the accuracy of the Treatment plan, relative to disease evolution and resulting toxicity levels that may develop during treatment. The RTTP module 126 also facilitates reviewing new or modified treatment plans, such as in cases where disease relapse has occurred or when it is necessary to change a treatment plan for other reasons. In addition, the RTTP module 126 records actual or true fraction doses acquired by a patient from treatment equipment and records the resulting treatment levels that a patient actually received.

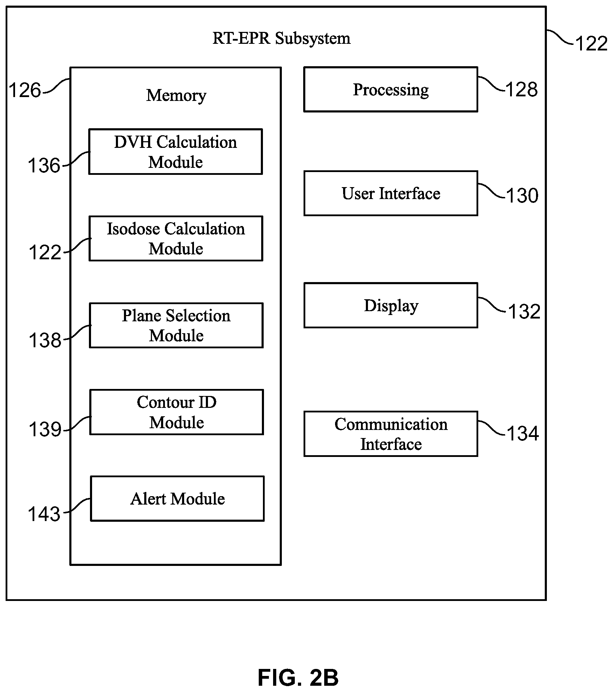

FIG. 2B illustrates an example block diagram of the RT-ERS subsystem 122 in accordance with embodiments herein. The RT-ERS subsystem 122 includes one or more memory 126, one or more processors 128, a user interface 130, a display 132 and one or more communications interfaces 134. The memory 126 includes program instructions, representing the RTTP module 126, directing the one or more processors 128 to implement a DVH calculation module 136, an isodose calculation module 137, a plane selection module 138, and a contour identification module 139.

The DVH calculation module 136 calculates dose volume histograms based on the dose information obtained from the dose objects and based upon anatomical structure information contained within the RT objects exported from the TPS. The isodose curve calculation module 137 calculates isodose curves relative to a set of planes of interest, such as the axial, sagittal and coronal planes.

The plane selection module 138 identifies the set of planes of interest to be utilized when calculating the isodose curves. In the present example, the plane selection module 138 has selected the axial, sagittal and coronal planes from a volumetric CT data set. Additionally or alternatively other sets of planes may be utilized where such alternative plane sets are oriented at a desired relation with respect to one another to provide multidimensional information regarding the anatomical structure of interest. The contour identification module 139 identifies the contours of one or more anatomical structures of interest within the set of selected planes.

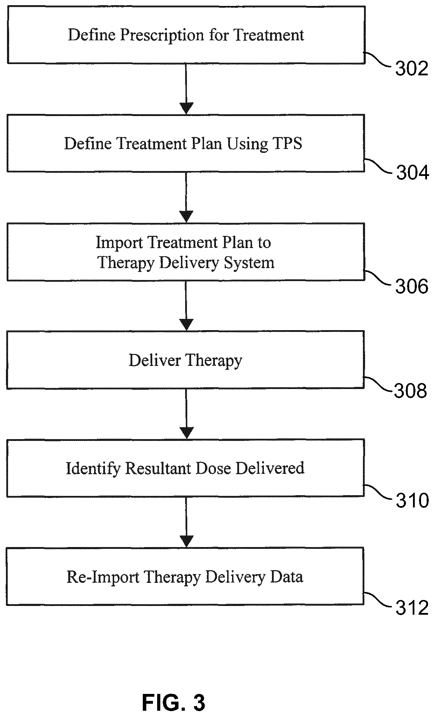

FIG. 3 illustrates an example overall radiotherapy workflow implemented in accordance with an embodiment herein. During operation, the workflow begins at 302 in an ambulatory phase where a prescription is defined for radiation treatment for an individual patient. FIG. 1A illustrates a more detailed example of the operations performed in connection with defining a prescription. The treatment includes the administration of a certain predetermined dose to one or more targets (denominated Clinical Target Volumes, CTV, or Planned Target Volumes, PTV). The treatment may include a predetermined number of sessions. Optionally, the sessions may be divided into sub-sessions referred to as fractions.

At 304, one or more of treatment plans are defined (e.g., concurrent plans or plans to be carried out at subsequent times) using a Treatment Planning System (e.g. TPS 150) based on the prescribed treatment. In various embodiments, multiple TPS may be utilized, wherein each TPS includes different aspects of some common features. For example, different TPS may be tailored to different types of procedures. The specific TPS is chosen depending on the specific needs of the patient (e.g., type of anatomy, nature of therapy to be delivered). A treatment plan, developed utilizing a TPS, is associated with a set of radiological studies that are used to identify the relevant anatomical structures. To define the treatment plan, images of the anatomical structures are displayed on the TPS, and the operator manually segments the structures using TPS software. In general, the TPS system includes one or more reference image series on which a treatment plan is defined. For example, the reference image series may be obtained by a CT apparatus, an MRI apparatus, a PET apparatus, etc.

When a CT apparatus is utilized, a CT reference image series is obtained by initially positioning the patient in the CT apparatus. The radiation therapy team takes reference computerized tomography (CT) images of the area of the body to be treated. The reference CT images are used to help the radiation oncologist determine the exact areas where treatment will be focused. To indicate the area where radiation should be aimed, the radiation therapy team uses a combination of laser lights and marks on the body. The lasers and marks also ensure that the patient's body is precisely aligned and positioned properly for each treatment session. The type of mark that the patient receives will depend on the type of cancer/tumor and the treatment. In certain cases, marks are drawn on the skin with a marker. Optionally, marks may be made that are permanent, such as small tattoos that represent permanent marks.

The treatment plan setup also defines the system settings for the radiation delivery system. For example, the setup may define the number of radiation beams, the geometry of the radiation beams and the radiation beam power levels. Based on the number, geometry and power, the treatment plan calculates a target dose to be hypothetically administered. The target dose is defined relative to a coordinate system of the TPS 150, such as a source Cartesian coordinate system where each coordinate represents a grid or lattice point within a range of a virtual radiation source within the coordinate system of the TPS 150. Each grid/lattice point is assigned a corresponding target radiation dose.

The TPS 150 allows the operator to verify the target doses that it is desired to be theoretically administered to the various anatomical structures as a function of the plane variation. The TPS 150 supports variation of the plan by the operator in order to reach a desired plane configuration that is intended to achieve the prescribed treatment.

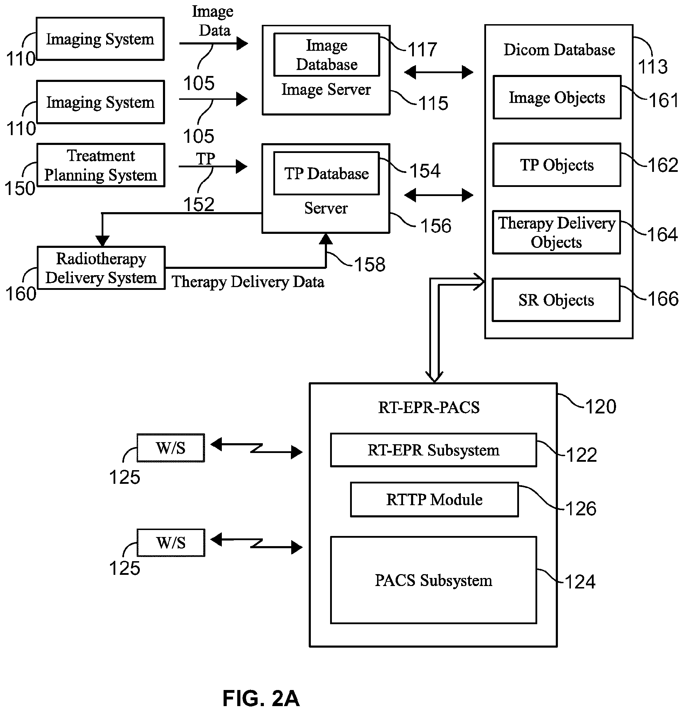

The treatment plan 152 is saved at 304, such as in a treatment plan database 154 (FIG. 2A). At 306, the treatment plan 152 is imported to the radiotherapy delivery system (e.g. 160 in FIG. 2A). At 308, a therapy is delivered based on the treatment plan 152 by the radiotherapy delivery system 160. At 310, the radiotherapy delivery system allows an operator to check the treatment once performed. For example, the radiotherapy delivery system may enable the operator to determine much dose has been effectively administered and in how many fractions. At 310, the radiotherapy delivery system 160 identifies a resulting dose that was delivered during the therapy. At 312, the radiotherapy delivery system 160 re-imports the therapy delivery data 158 to the treatment plan database 154. For example, the treatment delivery data 158 may include the beam configuration actually used. Optionally, the TPS system may then recalculate an actual spatial distribution of the administered doses and update the treatment plan saved in the database 154 and/or in the database 113.

The treatment plan 152 and the therapy delivery data 158 are conveyed to a DICOM database 113 as treatment plan objects 162 and therapy delivery objects 164 (at the time created, at any time prior to or during planning, at any time prior to or after therapy delivery). Optionally, the treatment planning database 154 may be removed entirely with the treatment plans and therapy delivery data conveyed directly to the database 113 as treatment plan objects and therapy delivery objects 162, 164.

### DICOM

The DICOM database 113 may store various types of information and data as objects in accordance with the DICOM standard. The Digital Imaging and Communication in Medicine ("DICOM") standard is maintained by the National Electronic Manufacturer's Association ("NEMA"), and is supported by image management systems, such as Picture Archive and Communication Systems ("PACS"). PACS systems are used in hospitals and labs to store, archive, .retrieve, search, and manage images used for clinical and research purposes in medicine, most typically for Radiology images such as radiography (e.g., X-Rays), computed tomography ("CT") scans, positron emission tomography ("PET"), and magnetic resonance imaging ("MRI"), but also for other modalities such as Ultrasonography, Cardiology, Endoscopy, and Mammography. A large number of clinical and laboratory instruments support DICOM-standard messaging as a means to communicate image information and store it in PACS systems. The DICOM standard is described further at:

http://dicom.nema.org/medical/Dicom/2015a/output/chtml/part03/PS3.3.html, the complete subject matter.

As described herein, image data files, treatment plans and therapy delivery data, among other things, are saved as DICOM objects. DICOM objects include numerous types of attributes. A DICOM attribute or data element is composed of i) A tag, in the format of group, element (XXXX,XXXX) that identifies the element, ii) A Value Representation (VR) that describes the data type and format of the attribute's value, iii) A value length that defines the length of the attribute's value and iv) A value field containing the attribute's data. For purposes of discussion, DICOM objects include image attributes, radio therapy attributes and dose attributes. At least a portion the RT modules within the DICOM standard are described at:

http://dicom.nema.org/medical/Dicom/2015a/output/chtml/part03/sect_C.8 .8.html, the complete subject matter.

Examples of image attributes (that are comprised within image object) are Image Area Dose Product, Image Box Content Sequence, Image Box Presentation LUT Flag, Image Center Point Coordinates Sequence, Image Comments, Image Display Format, Image Frame Origin, Image Horizontal Flip, Image ID, Image Index, Image Laterality, Image Orientation (Patient), Image Overlay Box Content Sequence, Image Overlay Flag, Image Plane Pixel Spacing, Image Position, Image Position (Patient), Image Rotation, Image Transformation Matrix, Image Translation Vector, Image Trigger Delay, Image Type, Imaged Nucleus, Imager Pixel Spacing, Images in Acquisition, Imaging Device-Specific Acquisition Parameters, Imaging Frequency, Imaging Service Request Comments,

Examples of radio therapy attributes (that are comprised within therapy delivery objects) include RT Beam Limiting Device Type, RT Dose ROI Sequence, RT Image Description, RT Image Label, RT Image, Name, RT Image Orientation, RT Image Plane, RT Image Position, RT Image SID, RT Plan Date, RT Plan, Description, RT Plan Geometry, RT Plan Label, RT Plan Name, RT Plan Relationship, RT Plan Time, RT Referenced Series Sequence, RT Referenced Study Sequence, RT Related ROI Sequence, RT ROI Identification Code, Sequence, RT ROI Interpreted Type, RT ROI Observations Sequence, RT ROI Relationship.

Examples of dose related attributes (that are comprised within treatment plan objects) include Dose Calibration Factor, Dose Comment, Dose Grid Scaling, Dose Rate Delivered, Dose Rate Set, Dose Reference Description, Dose Reference Number, Dose Reference Point Coordinates, Dose Reference Sequence, Dose Reference Structure Type, Dose Reference Type, Dose Summation Type, Dose Type, Dose Units, Dose Value, DVH Data, DVH Dose Scaling, DVH Maximum Dose, DVH Mean Dose, DVH Minimum Dose, DVH Normalization Dose Value, DVH Normalization Point, DVH Number of Bins, DVH Referenced ROI Sequence, DVH ROI Contribution Type, DVH Sequence, DVH Type, and DVH Volume Units.

It is understood that every image object, treatment plan object and therapy delivery object may not include each and every one of the above noted attributes. Instead, the foregoing attributes are examples provided in accordance with the DICOM standard.

Various combinations of the DICOM attributes may be utilized in connection with embodiments herein. For example, the dose attribute RT Dose IOD (see DICOM PS3.3 2015a - A.18) may be obtained to determine a distribution of doses calculated by the TPS. The focus for this Radiotherapy Dose IOD (RT Dose IOD) is to address the requirements for transfer of dose distributions calculated by radiotherapy treatment planning systems. These distributions may be represented as 2D or 3D grids, as isodose curves, or as named or unnamed dose points scattered throughout the volume. The RT Dose IOD may also contain dose-volume histogram data, single or multi-frame overlays and application-defined lookup tables. The dose distribution may be represented by grids, isodose curves or points. Optionally, the DICOM object may contain the DVH attribute, but this is not a required module. As another example of an RT attribute, the methods and systems herein access the RT Structure Set IOD (see DICOM PS3.3 2015a - A.19), which describes the patient anatomical structures defined as contours on the reference series of images. The RT Structure Set IOD addresses the requirements for transfer of patient structures and related data defined on CT scanners, virtual simulation workstations, treatment planning systems and similar devices. In general, the RT Structure Set information object defines a set of areas of interest (e.g. significance) in radiation therapy, such as body contours, tumor volumes (e.g. gross target volume, clinical target volume, planning target volume), OARs, and other ROIs. The target volumes may be defined in accordance with the guidelines in the International Commission on Radiation Units and Measurements Reports. For example, the gross target volume may generally represent the gross palpable, visible or clinical demonstrable extent and location of the tumor. The CTV contains the demonstrable gross target volume plus a margin for subclinical disease spread, cannot be fully imaged. The PPV is a geometric concept designed to ensure actual delivery of radiation therapy dose to the CTV and contains the CTV plus a margin to take into account uncertainties due to internal organ motion, patient motion and setup error. As one example, in the case of prostate cancer, the target volume represents the prostate gland and any parent glandular cancerous areas. The OARs are the urinary bladder, the rectum and the femoral heads. Each structure may be associated with a frame of reference, with or without reference to the diagnostic images.

The methods and systems herein also access RT Plan IOD (see DICOM PS3.3 2015a - A.20), which provides a definition of the treatment plan. The Radiotherapy Plan IOD (RT Plan IOD) addresses the requirements for transfer of treatment plans generated by manual entry, a virtual simulation system, or a treatment planning system before or during a course of treatment. Such plans may contain fractionation information, and define external beams and/or brachytherapy application setups. The RT Plan IOD may also indicate that the treatment utilizes treatment fractions to divide the treatment session.

### RT-EPR Evaluation

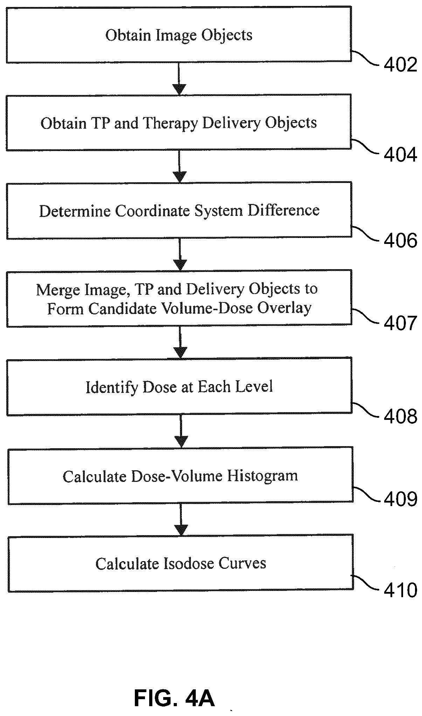

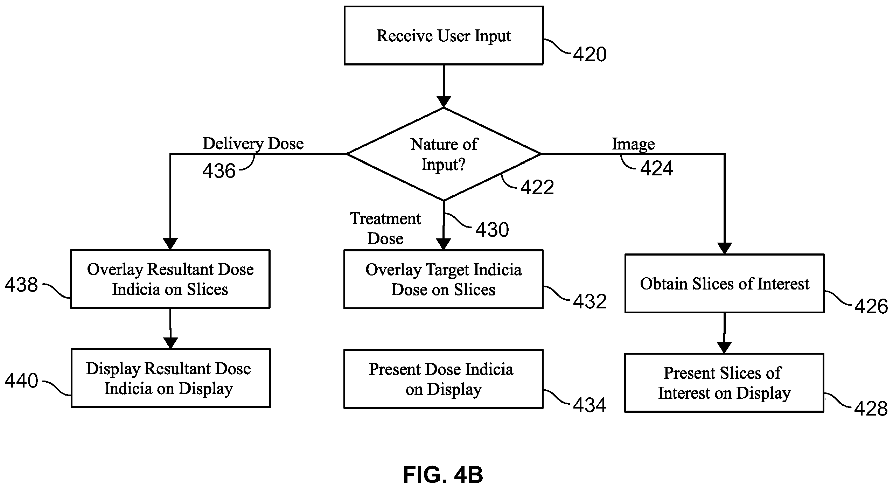

FIG. 4A illustrates a process carried out in accordance with embodiments herein to enable review and evaluation of treatment plans and resulting therapy delivery data. For example, the RT-EPR subsystem (FIG. 2A-2B) may implement the operations of FIG. 4 by directing one or more processors, through program instructions, to perform the following operations. The one or more processors may be implemented within a single or a combination of workstations, tablet devices, servers, smart phones and other processor-based electronic devices. In the embodiment of FIG. 4, it is assumed that the TPS 150 does not export a portion of the dose attributes with the treatment plan object. For example, the TPS 150 may not export the DVH and isodose curves with a treatment plan. Accordingly, many treatment plan objects 162 in the database 113 do not include DVH data and isodose curve data.

At 402, the processor(s) 128 (FIG. 2B) obtains one or more image objects form the database 113. The image objects include diagnostic image data 105 (FIG. 2A) that is defined relative to a coordinate system and resolution of the imaging system 110 acquired the image data 105. For example, a CT apparatus acquires a volumetric CT data set, and performs reconstruction to form a volumetric image data set. The image data set includes multiple CT image slices stacked adjacent to one another and/or a volume of voxels, the positions for which are defined relative to the coordinate system of the CT apparatus. The spacing between the voxels is defined by the resolution of the CT apparatus. As another example, when an MRI apparatus is used to acquire a volumetric MRI data set, the MRI apparatus performs reconstruction to form a volumetric image data set. The image data set includes multiple MRI image slices stacked adjacent to one another and/or a volume of voxels, the positions for which are defined relative to the coordinate system of the MRI apparatus. The spacing between the voxels is defined by the resolution of the MRI apparatus.

At 404, the processor(s) 128 obtains one or more RT objects, such as treatment plan objects 162 and therapy delivery objects 164 from the database 113. The RT objects (e.g., treatment plan and therapy delivery objects 162, 164) are defined by external planning systems, such as the treatment planning system 150 and the RDS 160. The RT objects include anatomical information regarding an anatomical structure of a treatment region and include dose data defining dose values for dose points in the treatment region. The dose points are misaligned with the voxels in the volumetric data set. The treatment plan objects 162 have a resolution and are defined relative to a coordinate system of the TPS 150. The therapy delivery objects 164 have a resolution and are defined relative to a coordinate system of the RDS 160. The resolution of the treatment plan objects 162 and therapy delivery objects 164 defines, among other things, the spacing between plan dose points and delivery dose points. The plan dose points in the treatment plan objects 162 have corresponding target dose values, while the delivery dose points in the therapy delivery objects 164 have corresponding resultant dose values.

At 406, the processor(s) 128 calculates a transformation to align the dose points with voxels in the volumetric data set. For example, the processors calculate a difference between the resolution and/or image coordinate system associated with the image objects 161 and the resolution and/or treatment coordinate system associated with the dose objects 162. Additionally or alternatively, the processor 128 may calculate a difference between the resolution and/or image coordinate system associated with the image objects 161 and the resolution and/or radiotherapy delivery coordinate system associated with the therapy delivery objects 164. As noted herein, the image coordinate system defines a volumetric reference grid that is associated with the image acquisition apparatus (e.g., a CT or MRI apparatus). The volumetric reference grid has a corresponding resolution (e.g., spacing between voxels). The resolutions of the image objects 161, dose objects 162, and therapy delivery objects 164 are referred to as image voxel resolution, target dose resolution and resultant dose resolution, respectively. The target dose resolution and/or resultant dose resolution do not coincide with the image voxel resolution. Additionally or alternatively, the coordinate system associated with the TPS 150 and/or RDS 160 do not coincide with the image coordinate system of the imaging system 110.

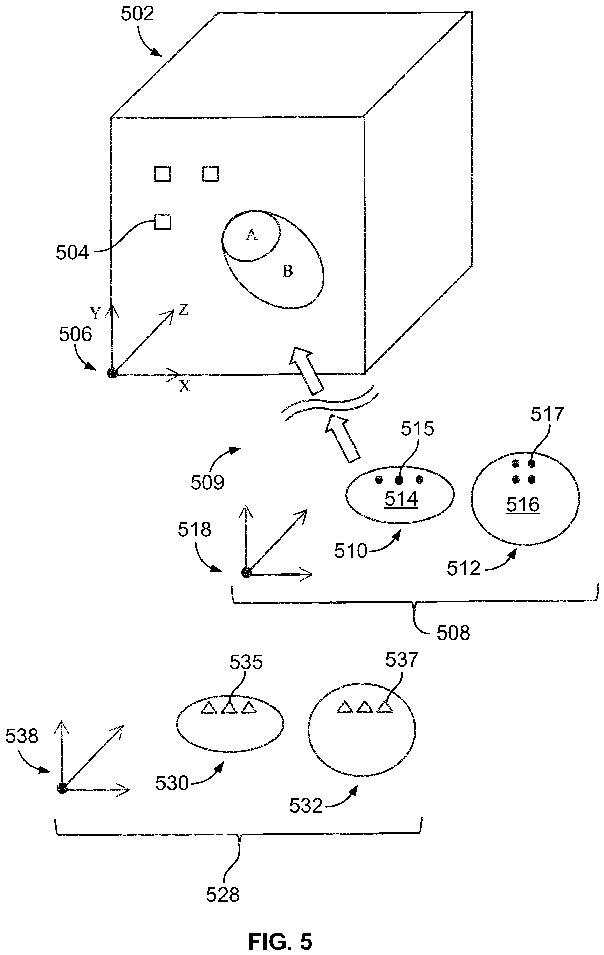

FIG. 5 illustrates an example of objects that may be operated upon in connection with the process of FIG. 4. In FIG. 5, an image object is illustrated as a volumetric data set 502 that includes a plurality of voxels 504 that are arranged relative to an image coordinate system 506. The image coordinate system 506 has a corresponding image voxel resolution that defines the spacing between the voxels 504. For convenience of illustration, the example of FIG. 5 is described in connection with two dimensions, however, it is understood that the same operations are performed in connection with a three dimensional system.

FIG. 5 also illustrates, as a treatment plan object, a structured data set 508 of structured objects 510, 512. Each of the structured objects 510, 512 has a corresponding target dose profile 514, 516 assigned thereto. Each of the structured objects 510, 512 is defined relative to a treatment plan coordinate system 518. For example, the structured data set 508 may define the shape of each structured object 510, 512, as well as a position and orientation relative to the treatment plan coordinate system 518. The treatment plan coordinate system 518 has a corresponding spacing between (and configuration of) plan dose points 515, 517, where each plan dose point 515, 517 has a corresponding target dose value defined by the treatment plan. The spacing of the plan dose points 515, 517 is referred to as target dose resolution.

The structured data set 508 may indicate, as part of a treatment plan, a baseline target dose profile 516 to be delivered to an area/volume within the boundaries defined by the structured object 512. The structured data set 508 may also include, as part of the treatment plan, an additional target dose profile 514 to be delivered to an area/volume within boundaries defined by the structured object 510. The shape, position and orientation of the structured objects 510, 512 are defined independent of, and separate from, any individual set of volumetric image data. Instead, the shape, position and orientation of the structured objects 510, 512 are defined relative to the treatment plan coordinate system 518.

FIG. 5 also illustrates, as a therapy delivery object, a structured data set 528 of structured objects 530, 532. Each of the structured objects 530, 532 has a corresponding target dose profile 534, 536 assigned thereto. Each of the structured objects 530, 532 is defined relative to a therapy deliver coordinate system 538. For example, the structured data set 528 may define the shape of each structured object 530, 532, as well as a position and orientation relative to the therapy delivery coordinate system 528.

The structured data set 528 may indicate that a resultant dose profile 536 was delivered to an area/volume within the boundaries defined by the structured object 532 as part of the therapy delivered. The structured data set 528 may also indicate that an additional resultant dose profile 534 is to be delivered to an area/volume within the boundaries defined by the structured object 530 as part of the therapy delivered. The shape, position and orientation of the structured objects 530, 532 are defined independent of and separate from any particular set of volumetric image data. Instead, the shape, position and orientation of the structured objects 530, 532 are defined relative to the coordinate system 538 of the radiotherapy delivery system. The structured data set 528 has a corresponding spacing between (and configuration of) delivery dose points 535, 537, where each delivery dose point 535, 537 have a corresponding resultant dose value for dose actually delivered during radio therapy. The spacing of the delivery dose points 535, 537 are referred to as resultant dose resolution.

With reference to FIGS. 4 and 5, at 406, the processor 128 identifies a resolution difference (e.g., mis-alignment) between the image voxel resolution, treatment dose resolution and delivery dose resolution, and calculates an adjustment corresponding to the resolution difference. Additionally or alternatively, the processor 128 identifies a difference (e.g., mis-alignment) between the imaging, therapy and treatment plan coordinate systems 506, 518, 528 and calculates an adjustment corresponding to the coordinate system difference. For example, the resolution difference may indicate that the spacing between image voxels is 80% of the spacing between target dose points. As another example, the resolution difference may indicate that the spacing between the image voxels (relative to the X-axis) is a first percentage difference from the spacing between target dose points, while the spacing between the image voxels (relative to the Y-axis) is a second percentage difference from the spacing between target dose points.

As another example, the coordinate system difference may indicate that the various coordinate systems 506, 518, 528 are translated relative to one another along one or more axes of a Cartesian coordinate system. Additionally or alternatively, the coordinate system difference may indicate that the various coordinate systems 506, 518, 528 are rotated relative to one another along one or more axes. As another example, the difference may indicate that one or more coordinate system is a Cartesian coordinate system, while one or more other coordinate system is a polar coordinate system. The processor 128 identifies a resolution and/or coordinate system transformation (generally noted at 509 in FIG. 5) that corresponds to the difference between the resolutions and/or coordinate systems.

For example, with reference to FIG. 5, the transformation 509 may indicate that the attributes (structured objects 510, 512) of the treatment plan object should be compressed, expanded rotated and/or translated by select degrees and/or distances along one or more of the X, Y, Y axes (e.g., apply a 2% compression along the X axis, apply a 4% expansion along the Y axis, shift 10 units left along the X axis and/or shift 8 units along the Y axis) to overlay treatment plan attribute data (structure objects 510, 512) onto the coordinate system of the volumetric data set 502 of the image object. Additionally or alternatively, the transformation 509 may indicate that the attributes of the treatment plan object should be rotates by select angles about one or more of the X, Y, Y axes to align treatment plan attribute data with the coordinate system of the image object. By applying a compression, expansion, translation and/or rotation, the target dose points (and/or delivery dose points) are matched to the voxels in the volumetric data set.

In the example of FIG. 5, the structured object 510 is transformed to overlay region A within the volumetric data set 502, while the structured object 512 is aligned to overlay both regions A and B in the volumetric data set 502, based on a common or different transformations 509. For example, the target dose points 515, 517 are aligned with the voxels 504. As one example, the combination of regions A and B may correspond to an organ, while region A may correspond to a tumor within or on the organ. A treatment plan may call for the entire organ (regions A and B) to be irradiated with a target baseline dose profile 516, corresponding to the area/volume within the boundaries of the structured object 512. In addition, it may be desirable to irradiate the tumor (region A) with a target additional dose profile 514, corresponding to the area/volume within the boundaries of a region defined by the structured object 510.

The structured object 530 is transformed to overlay region A of the volumetric data set 502, while the structured object 532 is aligned to overlay both regions A and B in the volumetric data set 502. The delivered therapy may have resulted in the entire organ (regions A and B) receiving a resultant baseline dose profile 536, corresponding to the area/volume within the boundaries of the structured object 532. In addition, the area/volume within the boundaries of the region defined by the structured object 530 received a resultant additional dose profile 534 which may differ from the target additional dose profile 514. The resultant baseline dose profile 536 and resultant additional dose profile 534 may differ from the target baseline dose profile 516 and target additional dose profile 514, respectively, for various reasons.

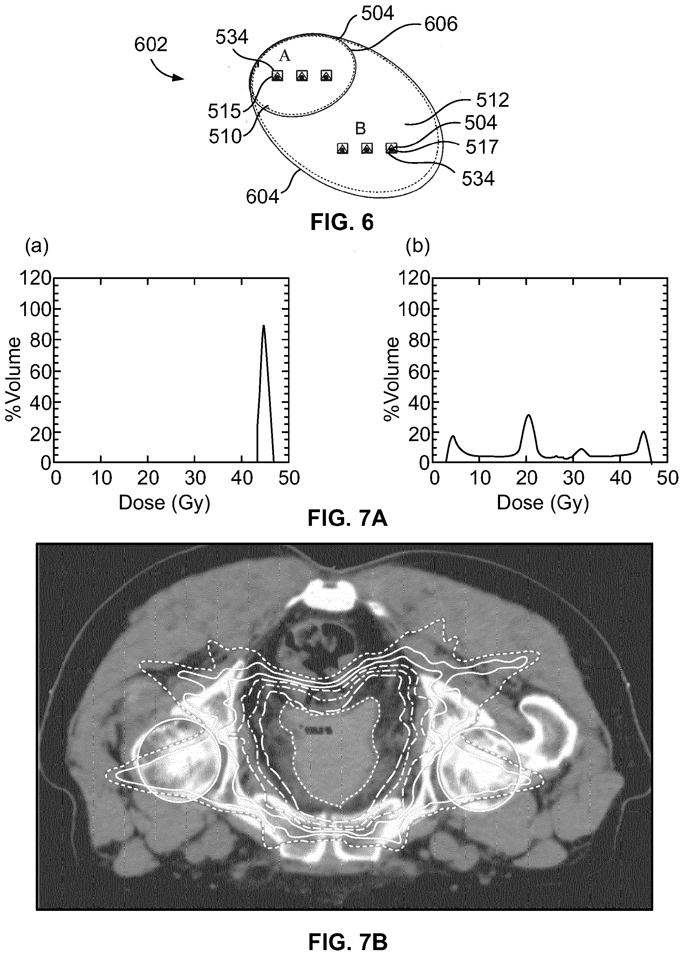

At 407, the treatment plan object is merged with one or more image objects based on the coordinate system transformation 509, to form a candidate volume-dose overlay (e.g., see FIG. 6). In the example of FIG. 5, the structured objects 510, 512 are overlaid upon the volumetric data set 502 to form the candidate volume-dose overlay. Additionally or alternatively, a therapy delivery object is merged with one or more image objects to form a candidate volumetric data set. For example, the structured objects 530, 532 are overlaid upon the volumetric data set 502 to form the candidate volume-dose overlay.

FIG. 6 illustrates an example candidate volume-dose overlay 602 that is formed after applying one or more resolution and/or coordinate system transformations 509 in accordance with embodiments herein. The overlay 602 corresponds to the example of FIG. 5. The organ of interest 604 corresponds to the combination of regions A and B, while the tumor 606 corresponds to region A. The boundaries of the organ 604 and tumor 606 are indicated in solid line. The overlay of the structured objects 510, 512 are indicated in dashed lines. The region within the boundary of the structured object 512 was planned to receive a target baseline dose as denoted by the wide crosshatching. The region with the boundary of the structured object 510 was planned to receive a target additional dose as denoted by the narrow crosshatching.

The resolution transformation 509 adjusts the spacing between the plan dose points 515 to match the spacing between the image voxels 504 (or vice versa). Additionally or alternatively, the resolution transformation 509 adjusts the spacing between the delivery dose points and the image voxels.

At 408, the processor 128 identifies (e.g., counts) the dose (target or resultant) received or to be received at each voxel in the volumetric data set. As is evident in FIG. 6, the boundaries of the structured objects 510, 512 have been transformed to substantially directly align with the boundaries of the organ 604 or tumor 606. The processor steps through each voxel 504 in the overlay 602 and identifies the corresponding target dose value to be received based on the treatment plan. It is recognized that only a portion of the voxels are indicated in FIG. 6. The voxels are analyzed for all or a portion of the volumetric data set and a corresponding treatment dose is recorded in connection with each voxel to form a voxel based dose estimate. Additionally or alternatively, the processor may also step through each voxel 504 and identify the corresponding delivered dose value that was delivered based on therapy delivery data.

The foregoing example illustrates structured objects that are intended to overlap one another. Additionally or alternatively, a treatment plan may define structured objects that do not overlap or abut against one another. In certain instances, one voxel may correspond to more than one structured object. When a voxel is overlaid by more than one structured object having a target dose associated therewith, the resulting dose is calculated by interpolation between the dose associated with each structured object. The result is then stored and optionally may be exported as a DICOM RT Dose IOD.

At 409, the processor(s) and DVH calculation module 136 calculate the Dose-Volume Histograms (DVH) data based on the voxel based dose estimates for the candidate volume-dose overlay 602. FIG. 7A illustrates differential DVH data for a four field prostate treatment plan for a target volume (a) and the rectum (b).

FIG. 7B a CT scan with superimposed dose indicia (color-coded) for radiation dose data generated in connection with a therapy planning. By way of example, the isodose curves may be shown in different colors (e.g. yellow, pink, green, magenta and blue). In addition, and RT structure set may be designated as a shaded area to indicate a region of interest, such as a tumor volume.

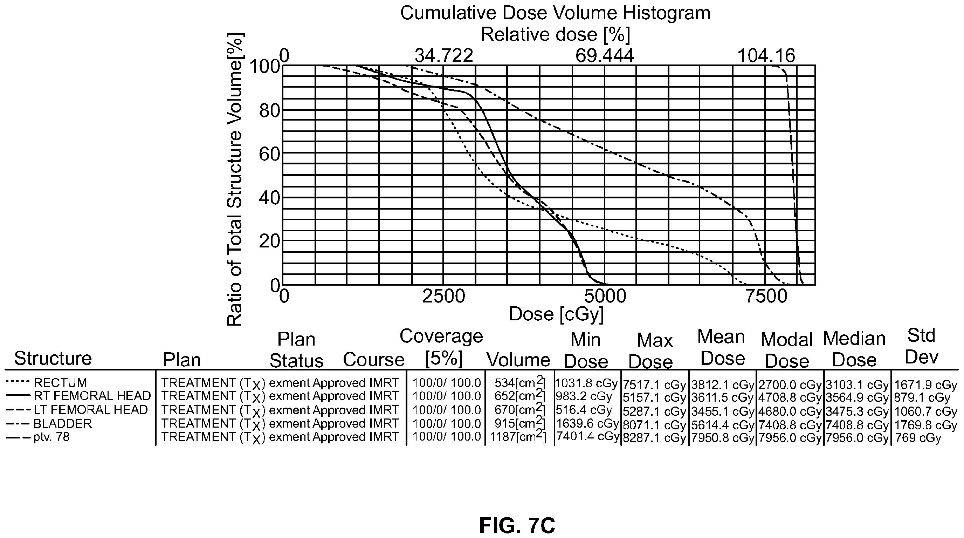

FIG. 7C illustrates a dose volume histogram corresponding to the information presented in FIG. 7B. The separate isodose curves within FIG. 7C corresponded to the colored lines in the image illustrated in FIG. 7B. By way of example, a red dose line may indicate that a majority of the tumor volume is receiving a select amount of radiation. In addition, the secondary dose lines (e.g. orange, green, magenta and yellow) indicate the dose received by various organs (e.g. the rectum, right femoral head, left femoral head and bladder, respectively, which correspond to OARs.

As one example, a collection of bins may be defined, where each bin is associated with a predetermined amount of dose. The DVH calculation module 136 may utilize a predetermined number of bins or bins having a predetermined amplitude. The bins can be of arbitrary size (e.g. 0-1 Gy, 1.001-2 Gy, 2.001-3 Gy, etc.). The histograms may be calculated in different manners. For example, a differential dose histogram may be calculated. Additionally or alternatively, a cumulative dose histogram may be calculated. When utilizing a differential dose histogram, the process counts the number of voxels having a dose within the bin range. The height of each bin is determined by the number of voxels whose dose is within the bin range. When utilizing cumulative dose histograms, the process counts a ratio between i) a number of voxels having a dose larger or equal to a dose threshold, and ii) a number of voxels having a dose below the dose threshold (also referred to as a bin limit). The height of each bin in a cumulative dose histogram is determined by the ratio of voxels above and below the dose threshold. The DVH calculation module 136 returns the DVH data as an RT Dose object (stream).

The operations at 409 and 410 may be performed serially in either order. Optionally, the operations at 409 410 may be performed in parallel.

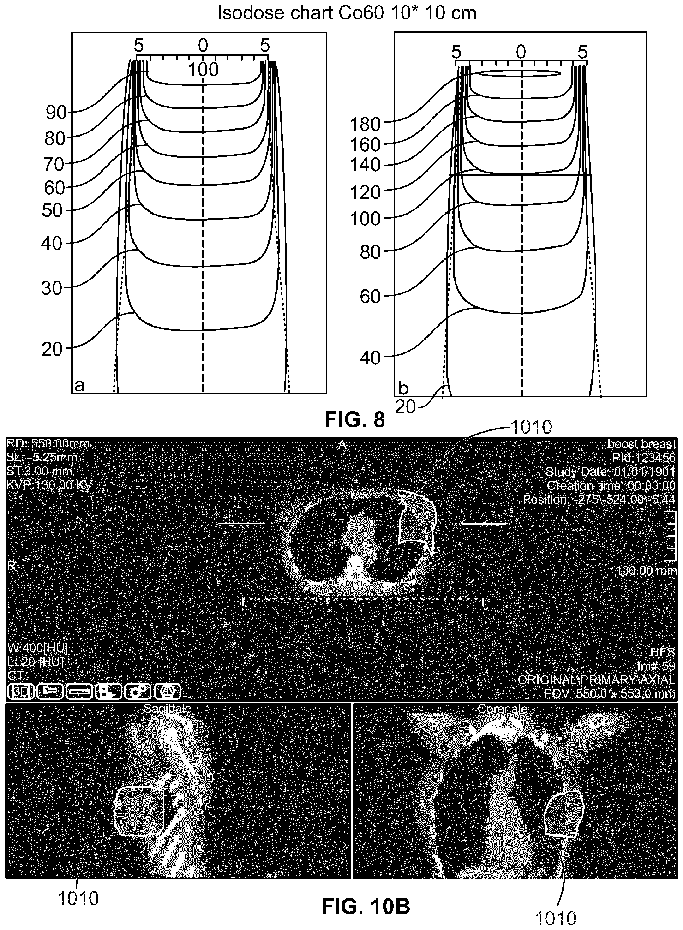

At 410, the processor(s) and isodose calculation module 137 calculate isodose curves based on the overlay 602 and the count of target dose to be received at each voxel. FIG. 8 illustrates isodose curves that may be calculated (e.g. by the isodose calculation module 137) in accordance with embodiments herein. The isodose curves represent the contours of the regions that receive a dose above a certain dose threshold. By way of example, the curves may be drawn at regular intervals of absorbed dose and expressed as percentages of the dose at a reference point. In FIG. 8, the isodose curves depict distributions of planar variation in expected absorbed dose, although isodose curves may depict distribution of dose in volumetric variation.

FIG. 4B illustrates a process for presenting treatment plan information to a user in accordance with embodiments herein. At 420, the processors 128 receive an input from a user. At 422, the processors 128 determine the nature of the input from the user. For example, the user may request various types of information. As one example, the input may indicate one or more image slices that the user desires to view, in which case, flow branches along 424. At 426, the processors obtain one or more slices of interest from the volumetric data set. For example, based on the user input, the plane selection module 138 identifies one or more plane of interest from the volumetric data set. The plane selection module 138 may initially identify an initial axial view through the volumetric data set. At 428 the slices of interest are displayed on the display 132 (FIG. 2B).

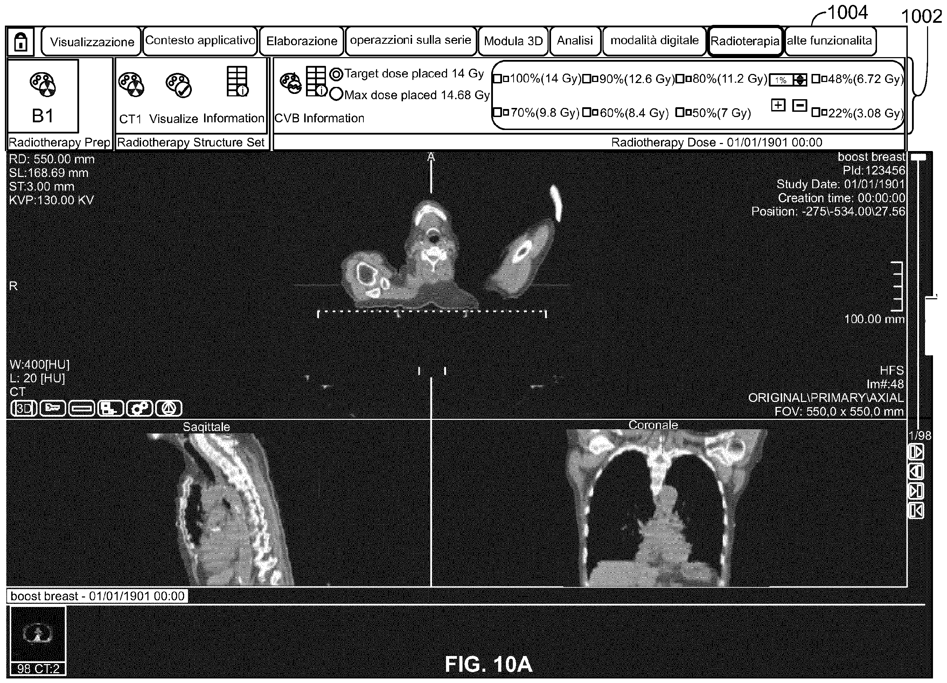

Returning to 422, the input from the user may indicate that the user desires to view dose information from a treatment plan, in which case, flow branches along 430. At 432, the processors overlay target dose indicia on one or more image slices that are displayed on the display. As one example, the target dose indicia maybe based on isodose curves that were previously calculated (e.g. at 410 in FIG. 4A). Alternatively, the indicia maybe based on isodose calculations that are performed relative to a particular slice or group of slices, where such calculation is performed at 432. At 434, the dose indicia are presented on the display. Examples of display formats for dose indicia are described hereafter in connection with FIGS. 10A-10D.

Returning to 422 in FIG. 4, the input from the user may indicate that the user desires to view dose information from radiotherapy that has already been delivered, in which case, flow branches along 436. At 438, the processors overlay resultant dose indicia on one or more slices that are being displayed on the display. As one example, the resultant dose indicia maybe based on isodose curves that were previously calculated (e.g. at 410 in FIG. 4A). Alternatively, the indicia maybe based on isodose calculations that are performed relative to a particular slice or group of slices, where such calculation is performed at 438. At 440, the dose indicia are presented on the display. Examples of formats in which dose indicia are displayed are described hereafter in connection with FIG. 10A-10D.

FIGS. 10A-10D illustrate screenshots of the manner in which image slices and dose indicia may be presented to a user in accordance with embodiments herein. FIG. 10A illustrates axial, sagittal and coronal views for image slices of a region of interest. The image slices may be manually selected by the user, or automatically determined by the processors 128 based on information in the treatment plan. During operation, the processor 128 may automatically select a set of views of interest, such as the axial, sagittal and coronal views. The user may utilize the automatically selected views or may manually select alternative views of interest. The processor 128 also automatically selects an initial image slice to be presented in connection with each view of interest. For example, the processor 128 may initially present, in connection with each view, a central slice through a center of a region of interest. Alternatively, the processor 128 may initially present, in connection with each view, a slice along one edge/side of the region of interest. The user may then manually adjust the slice(s) to view within the volumetric data set for the region of interest. For example, the user may manually step through a range of adjacent slices through the region of interest.

As the image slices are adjusted, the processor 128 automatically updates the dose indicia overlaid upon the currently presented set of image slices. For example, the dose indicia to be displayed may correspond to dose information of interest to the user. For example, the user may indicate an interest to view dose indicia corresponding to a dose range of interest (e.g., dose greater than a select level, dose less than a select level). The dose information of interest may be designated in various manners. For example, FIG. 10A illustrates a toolbar 1002 along one edge of the image with tabs 1004 that allow the user to enter additional information of interest (e.g., request to see particular dose information). As one example, the toolbar 1002 allows the user to select dose information of interest. For example, once the user enters dose information of interest, the processor 128 may overlay the image slices with dose indicia for regions that receive a certain amount of dose. As one example, the amount of dose may be designated as a percentage relative to a maximum dose (e.g., 100%, 90%, 80%, 70%, 60%, 50%). The dose percentage may be designated relative to a maximum dose (e.g., the real maximum value of dose points) or relative to the "target dose". For example, a target dose may be present in a dedicated DICOM tag or RT Plan IOD. The toolbar 1002 also allows the user to customize one or more particular amount of dose of interest.

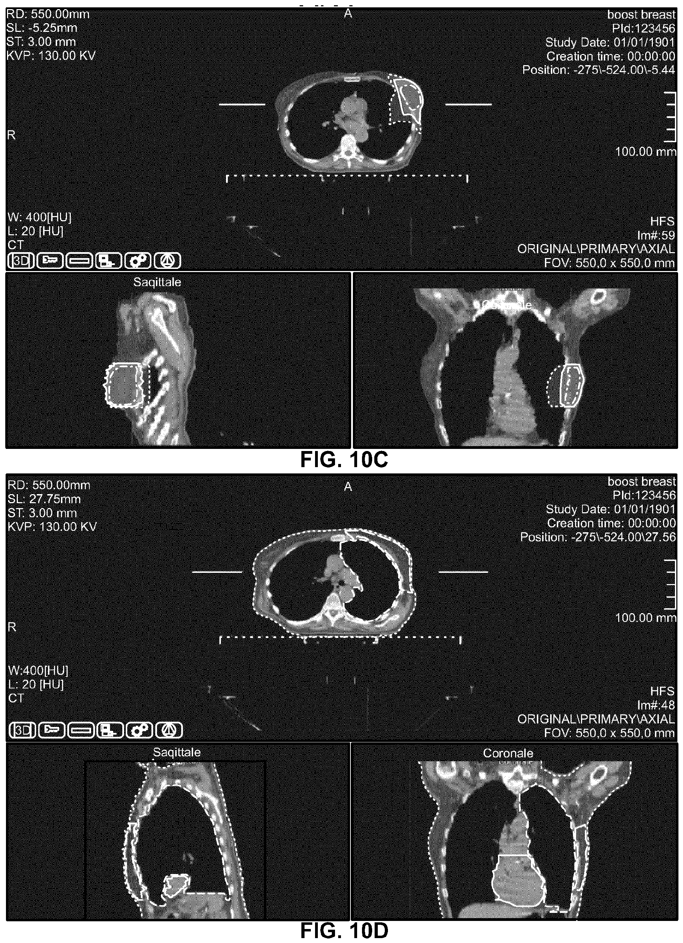

FIG. 10B illustrates a screenshot of images with dose indicia overlaid thereon, where the dose indicia correspond to a dose amount of interest to the user. In FIG. 10B, axial, coronal and sagittal views of image slices are displayed, along with dose indicia overlaid on the image slices. For example, an isodose contour 1010 may be overlaid upon a portion of one or more of the image slices. The isodose contour 1010 may be presented as a color-coded region, a shaded graphics or any other type of graphical information to identify the corresponding area. The isodose contour 1010 designates a region that receives a select amount of dose. For example, the isodose contour 1010 may indicate that, based on the treatment plan, the corresponding region is targeted to receive 22% of the target dose. It is recognized that other percentages may be designated. Optionally, multiple isodose contours may be designated, such as using multiple colors, different types of graphical information and the like. FIG. 10C illustrates a screenshot of images with dose indicia overlaid thereon. In the example of FIG. 10C different isodose curves are displayed. For example, one color (e.g., orange) represents the region that receive a first dose amount (e.g., more than 80% of the target dose), another color (e.g., yellow area/volume) represents the region that receives a second dose amount (e.g., 50%-80% of the target dose), while yet another color (e.g., blue area/volume) represents the region that receives a third dose amount (e.g., 22%-50%) of the target dose.

FIG. 10D illustrates a screenshot of images with RT structure outlines overlaid thereon. The RT structure outlines are extracted from the RT Structure Set IOD and displayed on one or more images. In the example of FIG. 10D, the images correspond to the three axial views (axial view, sagittal view, coronal view). The RT structure outlines may be presented on the image slices with or without the dose indicia overlaid thereon. As another example, a CT image slices may have superimposed thereon, color codes outlines to show an RT Structure Set, which includes tumor volume (e.g., outlined in red), OARs (e.g., femoral heads outlined in green, pink etc.), rectum (e.g., outlined in purple), bladder (e.g., outlined in blue), and body contour.

In accordance with the operations of FIG. 4A-4B, the isodose calculation module 137 calculates an isodose curve by identifying voxels that are scheduled to receive more than a certain dose threshold. For example, all or a portion of the voxels in the initial axial view are analyzed to identify the voxels having a corresponding common target dose (e.g., at least desired percentage of the target dose). As another example, a filter may be applied by the isodose calculation module 137 to identify neighboring voxels that exhibit dose transition within the initial axial view between predetermined percentages corresponding to the isodose curves. As another example, segmentation algorithms may be applied to identify the isodose curves for a corresponding dose levels.

In accordance with embodiments herein, multiple isodose curves (sets of isodose curves) are identified within the initial axial view, where each isodose curve corresponds to a different dose or percentage dose. Additionally or alternatively, multiple axial views/planes may be analyzed to identify corresponding sets of isodose curves.

As one example, the dose value for an associated isodose curve may be computed as a percentage of a maximum total dose (calculated automatically), or of the target dose provided as DICOM tag in the RT Plan IOD. The treatment plan may be defined based on standard orientation views (axial, sagittal, coronal) from an image set and being coincident with the acquisition grid of a CT apparatus. The isodose curves can be calculated for a single anatomical structure, for multiple anatomical structures or for all anatomical structures, starting from the dose information, the structures and the data set acquired by the CT apparatus. The isodose curve is returned as a binary image corresponding to the requested plane in which the isodose curve (and the region included if requested) has a value different from zero.

The processor(s) and contour identification module 139 identify contours of one or more anatomical structures of interest within select planes of interest from the image objects. For example, the planes of interest may represent the axial, coronal and sagittal views/planes. The contour identification module 139 calculates the contours for a given plane and for one or more anatomical structures. The plane may have only the standard orientation (e.g., axial, sagittal or coronal), and be coincident with the coordinate system of the imaging system that generated the volumetric data set (e.g. a CT apparatus). The contours are returned as images corresponding to the required level, with null values when there are no structures and values corresponding to referenced ROI Number (identification number of structures in the DICOM RT Structure) of the specified structure where a contour is present.

In accordance with embodiments herein, the processor(s) 128 extracts the planes of interest (e.g. sagittal and coronal planes) from a series of images acquired on a reference plane (e.g., the axial plane). For example, the user may enter an input to step through a series of image slices relative to one or more views/planes of interest. As the user selects a new image slice in one or more views, the processors 128 extract, from the volumetric data set, images corresponding to the slice and planes (e.g., standard orientation sagittal and coronal), and position coinciding with the coordinate system (acquisition grid) of the imaging system. The processor 128 calculates the corresponding dose indicia based on the isodose curves and presents the related image(s). The image(s) is returned with the same encoding of the input image.

Optionally, the DVHs and isodoses curves are stored in the DICOM database with the patient record corresponding to the image objects and TP object. The RT-EPR also displays the treatment plan and the analysis of the treatment plan on the same integrated workstation. The RT EPR enables the user to integrate the information calculated in order to evaluate the treatment plan and do statistical analysis.

Optionally, the RT-EPR subsystem 122 also includes an alerts module 143 that compares the planned/delivered doses with the protocol limit doses for the specific treatment. When the protocol limits are exceeded, visual alerts are provided to a user, such as through the display. Additionally or alternatively, the alerts module 143 may provide statistical analysis regarding effectiveness of treatments, toxicological effects, etc.

### TPS, Radiotherapy Delivery System and Imaging System

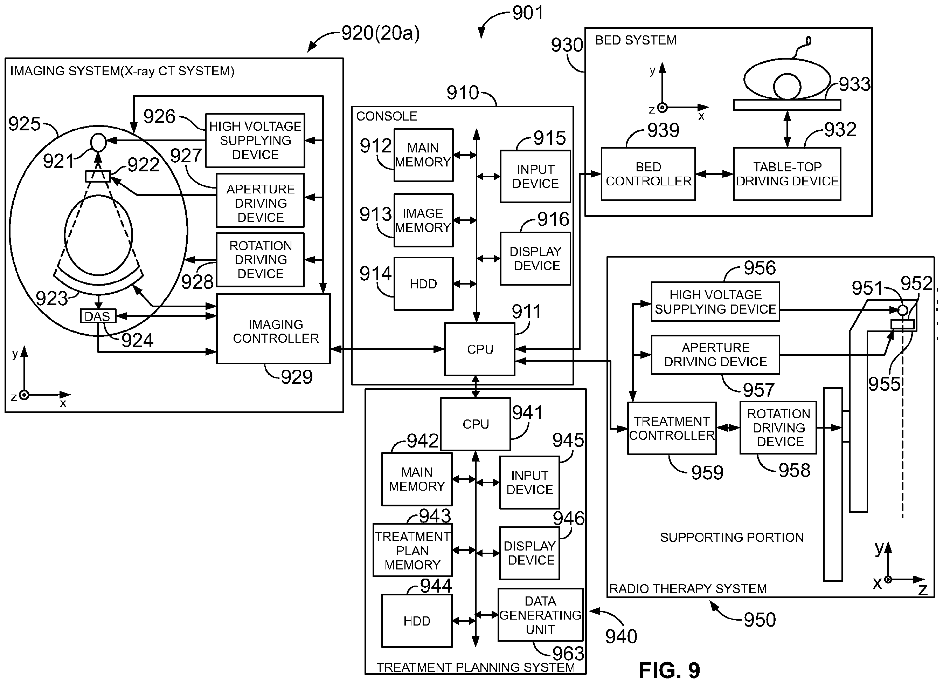

It is recognized that various configurations of TPS, radiotherapy delivery systems and imaging systems may be used to acquire the corresponding types of data. FIG. 9 illustrates one example of a TPS, radiotherapy delivery system and imaging system utilized in accordance with embodiments herein. A console 910 of the radiotherapy system 901 has a computer-based configuration and is able to communicate with a trunk network of a hospital such as a LAN (local area network), not shown. The console 910 is broadly composed of basic hardware such as a CPU (central processing unit) 911, main memory 912, image memory 913, an HDD (hard disc drive) 914, an input device 915, and a display device 916. The CPU 911 and each hardware component of the console 910 are connected with each other via buses as common signal transmission paths. In addition, the console 910 may include a recording medium drive.

The CPU 911 is a control device having a configuration of an integrated circuit (LSI) in which an electronic circuit composed of a semiconductor is housed in a package with multiple terminals. If an instruction is input by an operator such as a physician operating the input device 915, the CPU 911 executes a program stored in the main memory 912. Alternatively, the CPU 911 loads a program stored in the HDD 914, a program transferred from the network and installed in the HDD 914, or a program read out from a recording medium mounted in a recording medium drive (not shown) and installed in the HDD 914, into the main memory 912 to execute the program.

The main memory 912 is a storage device that includes ROM (read only memory), RAM (random access memory), or the like. The main memory 912 is used for storage of IPL (initial program loading), a BIOS (basic input/output system), and data. Also, the main memory 912 is used as working memory for the CPU 911 and to temporarily store data.

The image memory 913 is a storage device in which slice data as two-dimensional image data and treatment plan volume data and pretreatment volume data as three-dimensional image data are stored.

The HDD 914 is a storage device containing undetachable metal disks on which a magnetic substance is applied or evaporated. The HDD 914 is a storage device in which programs installed in the console 910 (in addition to application programs, including an OS (operating system)) and data are stored. In addition, the OS may be allowed to provide a GUI (graphical user interface) that makes heavy use of graphics in information displayed on the display device 916 so that an operator such as an operational person can perform basic operations through the input device 915.

The input device 915 is a pointing device that can be operated by the operator and input signals according to operations are sent to the CPU 911.

The display device 916 includes an image combining circuit, VRAM (video random access memory), and a display that are not shown. The image combining circuit generates composite data into which character data of a variety of parameters is combined with image data. The VRAM expands the composite data into display image data to be displayed on the display. The display is composed of a liquid crystal display, a CRT (cathode ray tube), or the like, and sequentially displays items of the display image data as display images.

The treatment planning system 940 of the radiotherapy system 901 generates treatment plan data for radiotherapy to be carried out by the radiotherapy system 950 on the basis of the slice data and the volume data generated by the console 910 after imaged by the X-ray CT system 920a. Under the control of the console 910 based on the treatment plan data generated by the treatment planning system 940, a site to be treated of the patient is irradiated by the radiotherapy system 950. The treatment planning system 940 has a computer-based configuration and can communicate with the trunk network of the hospital such as a LAN, not shown. The treatment planning system 940 is broadly composed of basic hardware such as a CPU 941, main memory 942, treatment plan memory 943, an HDD 944, an input device 945, and a display device 946. The CPU 941 and each hardware component of the treatment planning system 940 are connected with each other via buses as common signal transmission paths. In addition, the treatment planning

A configuration of the CPU 941 is equivalent to that of the CPU 911 of the console 910. If the operator operates the input device 945 to input an instruction, the CPU 941 executes a program stored in the main memory 942. Alternatively, the CPU 941 loads a program stored in the HDD 944, a program transferred from the network and installed in the HDD 944, or a program read out from a recording medium mounted in a recording medium drive (not shown) and installed in the HDD 944, into the main memory 942 to execute the program.

A configuration of the main memory 942 is equivalent to that of the main memory 912 of the console 910. The main memory 942 is used for storage of IPL, a BIOS, and data. Also, the main memory 942 is used as working memory for the CPU 941 and to temporarily store data. The treatment plan memory 943 is a storage device in which treatment plan data is stored.

The treatment planning system 940 determines a position of the treatment site of the patient and a shape of the treatment site based on the image data generated by the X-ray CT system 920a, and also determines a type of radiation (an X-ray, an electron beam, a neutron beam, a proton beam, a heavy particle beam, or the like) to be applied to the treatment site, energy of the radiation, and a radiation field.

The treatment plan data generating unit 963 of the treatment planning system 940 shown in FIG. 3 has a function of setting a treatment plan by setting irradiation conditions such as a direction and number of irradiation, and a radiation intensity with a contour of a body of the patient and a region of an affected part taken into consideration based on the treatment plan volume data stored in the image memory 913, to generate treatment plan data. When generating the treatment plan data, the treatment plan data generating unit 963 sets, on the basis of the treatment plan volume data, a required area, for example, a contour of an OAR (organ at risk) not to be irradiated. For example, the treatment plan data generating unit 963 sets the contour of the OAR through the input device 945. The contour of the OAR set by the treatment plan data generating unit 963 is three-dimensional position information. When setting a contour of an OAR, the treatment plan data generating unit 963 may set a contour SP1 of only one OAR or may set contours SP1 SP2, . . . of a plurality of OARs. In addition, when generating the treatment plan data, the treatment plan data generating unit 963 may set a comparison point (isocenter). FIG. 6 is a diagram schematically showing an example of a display image of a contour of an OAR based on the treatment plan volume data.

Next, a first operation of the radiotherapy system 901 of the first embodiment will be described. If the patient is placed on the table-top 933 of the bed system 930 of the radiotherapy system 901, the radiotherapy system 901 controls the operations of the bed controller 939 of the bed system 930 to insert the table-top 933 into an opening of the X-ray CT system 920a. Then, as shown in FIG. 9, the radiotherapy system 901 controls the operations of the imaging controller 929 of the X-ray CT system 920a to image a region including the treatment site of the patient for treatment planning. Then, the radiotherapy system 901 performs processing such as image reconstructing processing on transmission data obtained by the X-ray CT system 920a to generate slice data as two-dimensional image data, and generates treatment plan volume data as three-dimensional image data based on the slice data corresponding to a plurality of slices. The treatment plan volume data generated is stored in a storage device such as the image memory 913.

The radiotherapy system 901 sets a treatment plan by setting irradiation conditions such as a direction and number of irradiation, and a radiation intensity with a contour of a body of the patient and a region of an affected part taken into consideration based on the treatment plan volume data stored in the image memory 913, to generate treatment plan data. The radiotherapy system 901 sets a contour of an OAR as a required region based on the treatment plan volume data. The radiotherapy system 901 also computes a DV histogram of the contour of the OAR set. The treatment plan data generated is stored in a storage device such as the treatment plan memory 943.

When the imaging of the region including the treatment site of the patient ends, the radiotherapy system 901 controls the operations of the bed controller 939 of the bed system 930 to retreat the table-top 933 from the opening of the X-ray CT system 920a. Then, the patient is removed from the table-top 933 of the bed system 930 of the radiotherapy system 901.

If the patient is placed on the table-top 933 of the bed system 930 of the radiotherapy system 901 just before the treatment is performed by the radiotherapy system 950, the radiotherapy system 901 controls the operations of the bed controller 939 of the bed system 930 to insert the table-top 933 into the opening of the X-ray CT system 920a. Then, as shown in FIG. 9, the radiotherapy system 901 controls the operations of the imaging controller 929 of the X-ray CT system 920a to image the region including the treatment site of the patient just before the treatment. Then, the radiotherapy system 901 performs processing such as image reconstructing processing on transmission data obtained by the X-ray CT system 920a to generate slice data as two-dimensional image data, and generates pretreatment volume data as three-dimensional image data based on the slice data corresponding to a plurality of slices. The pretreatment volume data generated is stored in a storage device such as the image memory 913.

The radiotherapy system 901 sets a contour of the OAR corresponding to the contour of the OAR stored in the treatment plan memory 943 based on the pretreatment volume data stored in the image memory 913. The radiotherapy system 901 also computes a DV histogram of the contour of the OAR based on the treatment plan set and the contour of the OAR set.

Then, the radiotherapy system 901 computes differences D between doses in the same volumes based on the DV histogram of the contour of the OAR set in FIG. 9 and the DV histogram of the contour of the OAR set.

Then, the radiotherapy system 901 determines whether or not a maximum difference Dmax of the difference D in each of the volumes computed is equal to or smaller than a threshold value. If yes, that is, if it is determined that the maximum difference Dmax of the difference D in each of the volumes is equal to or smaller than the threshold value, the radiotherapy system 901 allows for processing in a next step.

Then, the radiotherapy system 901 controls the operations of the treatment controller 959 of the radiotherapy system 950 to treat the treatment site of the patient. After the treatment site of the patient is treated, the radiotherapy system 901 controls the operations of the bed controller 939 of the bed system 930 to retreat the table-top 933 from the radiotherapy system 950. Then, the patient is removed from the table-top 933 of the bed system 930 of the radiotherapy system 901.

On the other hand, if no, that is, if it is determined that the maximum difference Dmax of the difference D in each of the volumes is greater than the threshold value, the radiotherapy system 901 reports an abnormality to the operator., For example, the radiotherapy system 901 reports an abnormality to the operator through the display device 916. Then, after a setting is carried out again by shifting the patient on the table-top, the radiotherapy system 901 performs the pretreatment imaging.

If the radiotherapy system 901 reports the abnormality to the operator, the treatment planning system 940 reconsiders the treatment plan set and sets a treatment plan again. For example, with the treatment plan set as an initial setting, the irradiation conditions such as a direction and number of irradiation, and radiation intensity are set again. Then, the radiotherapy system 901 computes a DV histogram of the contour of the OAR based on the treatment plan set again and the contour of the OAR set.

In addition, the radiotherapy system 901 may also compute the DV histogram of the contour of the OAR associated with the irradiation conditions simultaneously as the irradiation conditions are set again to immediately display the DV histogram. In this case, it is suitable to display the DV histogram computed with the DV histogram, which is immediately displayed. The operator can judge suitability by comparing the immediately displayed DV histogram with the DV histogram.

According to the radiotherapy system 901 of the first embodiment, the DV histogram of the contour of the OAR or the like included in the treatment plan volume data is compared with the DV histogram of the contour of the OAR or the like included in the pretreatment volume data, and if a difference therebetween is significant, the fact that a setting of the patient is required again to carry out treatment based on a treatment plan can be reported to the operator.

### Conclusions

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

Aspects are described herein with reference to the Figures, which illustrate example methods, devices and program products. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

One or more of the operations described above in connection with the methods may be performed using one or more processors. The different devices in the systems described herein may represent one or more processors, and two or more of these devices may include at least one of the same processors. In one embodiment, the operations described herein may represent actions performed when one or more processors (e.g., of the devices described herein) execute program instructions stored in memory (for example, software stored on a tangible and non-transitory computer readable storage medium, such as a computer hard drive, ROM, RAM, or the like).

The processor(s) may execute a set of instructions that are stored in one or more storage elements, in order to process data. The storage elements may also store data or other information as desired or needed. The storage element may be in the form of an information source or a physical memory element within the controllers and the controller device. The set of instructions may include various commands that instruct the controllers and the controller device to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

The controller may include any processor-based or microprocessor-based system including systems using microcontrollers, reduced instruction set computers (RISC), application specific integrated circuitry (ASICs), field-programmable gate arrays (FPGAs), logic circuitry, and any other circuit or processor capable of executing the functions described herein. When processor-based, the controller executes program instructions stored in memory to perform the corresponding operations. Additionally or alternatively, the controllers and the controller device may represent circuitry that may be implemented as hardware. The above examples are exemplary only, and are thus not intended to limit in any way the definition and/or meaning of the term "controller."

Optionally, aspects of the processes described herein may be performed over one or more networks one a network server. The network may support communications using any of a variety of commercially-available protocols, such as Transmission Control Protocol/Internet Protocol ("TCP/IP"), User Datagram Protocol ("UDP"), protocols operating in various layers of the Open System Interconnection ("OSI") model, File Transfer Protocol ("FTP"), Universal Plug and Play ("UpnP"), Network File System ("NFS"), Common Internet File System ("CIFS") and AppleTalk. The network can be, for example, a local area network, a wide-area network, a virtual private network, the Internet, an intranet, an extranet, a public switched telephone network, an infrared network, a wireless network, a satellite network and any combination thereof.

In embodiments utilizing a web server, the web server can run any of a variety of server or mid-tier applications, including Hypertext Transfer Protocol ("HTTP") servers, FTP servers, Common Gateway Interface ("CGI") servers, data servers, Java servers, Apache servers and business application servers. The server(s) also may be capable of executing programs or scripts in response to requests from user devices, such as by executing one or more web applications that may be implemented as one or more scripts or programs written in any programming language, such as Java®, C, C# or C++, or any scripting language, such as Ruby, PHP, Perl, Python or TCL, as well as combinations thereof. The server(s) may also include database servers, including without limitation those commercially available from Oracle®, Microsoft®, Sybase® and IBM® as well as open-source servers such as MySQL, Postgres, SQLite, MongoDB, and any other server capable of storing, retrieving and accessing structured or unstructured data. Database servers may include table-based servers, document-based servers, unstructured servers, relational servers, non-relational servers or combinations of these and/or other database servers.

The embodiments described herein may include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers or other network devices may be stored locally and/or remotely, as appropriate. Where a system includes computerized devices, each such device can include hardware elements that may be electrically coupled via a bus, the elements including, for example, at least one central processing unit ("CPU" or "processor"), at least one input device (e.g., a mouse, keyboard, controller, touch screen or keypad) and at least one output device (e.g., a display device, printer or speaker). Such a system may also include one or more storage devices, such as disk drives, optical storage devices and solid-state storage devices such as random access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.) and working memory as described above. The computer-readable storage media reader can be connected with, or configured to receive, a computer-readable storage medium, representing remote, local, fixed and/or removable storage devices as well as storage media for temporarily and/or more permanently containing, storing, transmitting and retrieving computer-readable information. The system and various devices also typically will include a number of software applications, modules, services or other elements located within at least one working memory device, including an operating system and application programs, such as a client application or web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Various embodiments may further include receiving, sending, or storing instructions and/or data implemented in accordance with the foregoing description upon a computer-readable medium. Storage media and computer readable media for containing code, or portions of code, can include any appropriate media known or used in the art, including storage media and communication media, such as, but not limited to, volatile and non-volatile, removable and non-removable media implemented in any method or technology for storage and/or transmission of information such as computer readable instructions, data structures, program modules or other data, including RAM, ROM, Electrically Erasable Programmable Read-Only Memory ("EEPROM"), flash memory or other memory technology, Compact Disc Read-Only Memory ("CD-ROM"), digital versatile disk (DVD) or other optical storage, magnetic cassettes, magnetic tape, magnetic disk storage or other magnetic storage devices or any other medium which can be used to store the desired information and which can be accessed by the system device. Based on the disclosure and teachings provided herein, a person of ordinary skill in the art will appreciate other ways and/or methods to implement the various embodiments.

The specification and drawings are, accordingly, to be regarded in an illustrative rather than a restrictive sense. It will, however, be evident that various modifications and changes may be made.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including" and "containing" are to be construed as open-ended terms (i.e., meaning "including, but not limited to,") unless otherwise noted. The term "connected," when unmodified and referring to physical connections, is to be construed as partly or wholly contained within, attached to or joined together, even if there is something intervening. Recitation of ranges of values herein are merely intended to serve as a shorthand method of referring individually to each separate value falling within the range, unless otherwise indicated herein and each separate value is incorporated into the specification as if it were individually recited herein. The use of the term "set" (e.g., "a set of items") or "subset" unless otherwise noted or contradicted by context, is to be construed as a nonempty collection comprising one or more members. Further, unless otherwise noted or contradicted by context, the term "subset" of a corresponding set does not necessarily denote a proper subset of the corresponding set, but the subset and the corresponding set may be equal.

Operations of processes described herein can be performed in any suitable order unless otherwise indicated herein or otherwise clearly contradicted by context. Processes described herein (or variations and/or combinations thereof) may be performed under the control of one or more computer systems configured with executable instructions and may be implemented as code (e.g., executable instructions, one or more computer programs or one or more applications) executing collectively on one or more processors, by hardware or combinations thereof. The code may be stored on a computer-readable storage medium, for example, in the form of a computer program comprising a plurality of instructions executable by one or more processors. The computer-readable storage medium may be non-transitory.

Preferred embodiments of this disclosure are described herein, including the best mode known to the inventors for carrying out the invention. Variations of those preferred embodiments may become apparent to those of ordinary skill in the art upon reading the foregoing description. The inventors expect skilled artisans to employ such variations as appropriate and the inventors intend for embodiments of the present disclosure to be practiced otherwise than as specifically described herein. Accordingly, the scope of the present disclosure includes all modifications and equivalents of the subject matter recited in the claims appended hereto as permitted by applicable law. Moreover, any combination of the above-described elements in all possible variations thereof is encompassed by the scope of the present disclosure unless otherwise indicated herein or otherwise clearly contradicted by context.

## Claims

1. A method for providing radiotherapy workflow management relating to a treatment plan based on standard orientation views (axial, sagittal, coronal) at a radiotherapy electronic patient record (RT-EPR) system integrated with a picture archive computing system (PACS), the method comprising:
Obtaining an image object that includes a volumetric data set of voxels having a spacing and position relative to an image coordinate system;
obtaining Dicom RT objects that include anatomical information regarding an anatomical structure of a treatment region (RT Structure) and including dose data (RT Dose) defining dose values for dose points in the treatment region, wherein the dose points are misaligned with the voxels of the volumetric data set,
**characterized in**:
calculating a transformation to align the dose points with voxels in the volumetric data set;
calculating at least one isodose curve based on the volumetric data set and the dose values;
displaying, through a workstation viewer, axial, coronal, sagittal views of image slices from the volumetric data set with dose indicia superimposed on the image slices, the dose indicia, indicative of the dose data and based on the isodose curve, comprising a contour (1010) designating a region that receives a select amount of dose.

2. The method of claim 1, further comprising presenting the dose indicia for treatment regions having dose values greater than a select dose value.

3. The method according to claim 1 or 2, wherein the dose points represent one or more of:
i) plan dose points in a treatment plan, the plan dose points and voxels having different first and second resolutions such that at least a portion of the dose points are misaligned with the voxels in the volumetric data set, the transformation aligning the plan dose points and the voxels; or
ii) delivery dose points from a radiotherapy delivery system, the delivery dose points and voxels having different first and second resolutions, the transformation aligning the delivery dose points and voxels.

4. The method according to any preceding claim, wherein the transformation includes at least one of i) changing a target dose resolution of the dose points, and ii) performing interpolation between the dose points to align with an image voxel resolution of the volumetric data set with the dose points.

5. The method according to any preceding claim, wherein the RT objects include one or more of:
i) a first set of RT objects that includes the anatomical information and a second set of the RT objects that includes the dose data; or
ii) a set of DICOM RT objects generated by a therapy planning system, the set of DICOM RT objects representing at least one of: i) the external system, the set of DICOM RT objects including information about the anatomical structure of the treatment region defined relative to the image coordinate system of the volumetric data set, or ii) information about the planned doses for a plurality of points distributed throughout the treatment region, where at least a portion of the points are misaligned with the voxels in the volumetric data set; or
iii) a set of RT objects generated by or more external planning systems, the obtaining operation including accessing a DICOM database that stores the RT objects.

6. The method according to any preceding claim, wherein the displaying includes displaying the image slices along axial, coronal and sagittal views planes, with the dose indicia overlaid thereon.

7. The method according to any preceding claim, where the calculating and displaying operations are launched by a RT-EPR system that collects clinical information about a patient status and a patient care plan.

8. The method according to any preceding claim, further:
calculating a dose volumetric histogram (DVH) data based on the volumetric data set and the dose values and
displaying, through the workstation viewer, image slices from the volumetric data set with dose indicia indicative of the dose data superimposed on the image slices, the dose indicia being based on DVH data.

9. A radiotherapy electronic patient record (RT-EPR) system integrated with a picture archive computing system (PACS) for providing radiotherapy workflow management relating to a treatment plan based on standard orientation views (axial, sagittal, coronal), the system comprising:
a workstation;
a communications interface configured to obtain an image object that includes a volumetric data set of voxels having a spacing and position relative to an image coordinate system;
the communications interface configured to obtain RT objects that include anatomical information regarding an anatomical structure (RT Structure) of a treatment region and include dose data (RT Data) defining dose values for dose points in the treatment region, the dose points misaligned with the voxels in the volumetric data set;
memory storing program instructions,
**characterized in** comprising a processor configured to execute the program instructions to:
calculate a transformation to align the dose points with voxels in the volumetric data set;
calculate at least one isodose curve based on the volumetric data set and the dose values; and
direct the workstation to display axial, coronal, sagittal views of image slices from the volumetric data set with dose indicia superimposed on the image slices, the dose indicia, indicative of the dose data and based on the isodose curve, comprising a contour (1010) designating a region that receives a select amount of dose.

10. The system of claim 9, wherein the processing is further configured to perform one or more of:
i) presenting the dose indicia for treatment regions having dose values greater than a select dose value;
ii) representing the dose points as plan dose points in a treatment plan, the plan dose points and voxels having different first and second resolutions, the transformation aligning the plan dose points and voxels; or
iii) representing the dose points as delivery dose points from a radiotherapy delivery system, the delivery dose points and voxels having different first and second resolutions, the transformation aligning the delivery dose points and voxels.

11. The system according to claim 9 or 10, wherein the transformation includes changing a target dose resolution of the dose points to align with an image voxel resolution of the volumetric data set.

12. The system according to any preceding claim 9 to 11, wherein a first set of RT objects includes the anatomical information and a second set of the RT objects includes the dose data.

13. The system according to any preceding claim 9 to 12, wherein the RT objects include a set of DICOM RT objects generated by a therapy planning system, the set of DICOM RT objects representing the external system, the set of DICOM RT objects including information about the anatomical structure of the treatment region defined relative to the image coordinate system of the volumetric data set.

14. The system according to any preceding claim 9 to 13, wherein the RT objects include a set of DICOM RT objects generated by a therapy planning system, the set of DICOM RT objects representing information about the planned doses for a plurality of points distributed throughout the treatment region, where at least a portion of the points are misaligned with the voxels in the volumetric data set.

15. The system according to any preceding claim 9 to 14, wherein the displaying includes displaying the image slices along axial, coronal and sagittal views planes, with the dose indicial overlaid thereon.

16. The system according to any preceding claim 9 to 15, further comprising a DICOM database that stores the RT objects and the image objects.

## Patentansprüche

1. Eine Methode zur Bereitstellung einer Radiotherapie-Workflow-Verwaltung in Bezug auf einen Therapieplan, der auf standardmäßigen Ausrichtungsorientierungen (axial, sagittal, koronal) eines computergesteuerten Patientenaktensystems beruht (RT-EPR = RadioTherapy Electronic Patient Record) und ein Bildarchiv-Computersystem beinhaltet (PACS = Picture Archive Computing System). Die Methode schließt Folgendes ein:
Erhalt eines Bildobjekts, das einen volumetrischen Voxel-Datensatz beinhaltet, der Abstände und Positionen in Bezug auf ein Bild-Koordinatensystem zur Verfügung stellt;
Erhalt von Dicom RT Objekten, die anatomische Informationen hinsichtlich der anatomischen Struktur eines Behandlungsbereichs (RT Struktur) beinhalten und Dosierungsdaten (RT Dosierung) einschließen, die Dosierungswerte für Dosierungspunkte im Behandlungsbereich bestimmen, wobei die Dosierungspunkte hinsichtlich der Voxel des volumetrischen Datensatzes verschoben sind,
**gekennzeichnet durch**:
Berechnung einer Transformation zum Ausrichten der Dosierungspunkte mit Voxel im volumetrischen Datensatz;
Berechnung mindestens einer Isodosenkurve beruhend auf dem volumetrischen Datensatz und den Dosierungspunkten;
Wiedergabe von axialen, koronalen, sagittalen Ansichten von Bildausschnitten des volumetrischen Datensatzes mit Hilfe einer Viewer-Workstation, wobei Dosierungsmarkierungen in die Bildausschnitte eingeblendet werden. Die Dosierungsmarkierungen zeigen die Dosierungsdaten auf der Grundlage der Isodosenkurve an und umfassen eine Konturführung (1010) zur Festlegung eines Bereichs, der eine bestimmte Dosierungsmenge erhält.

2. Verfahren nach Anspruch 1, das darüberhinaus die Dosierungsmarkierungen für Behandlungsbereiche, die höhere Dosierungswerte als ein ausgewählter Dosierungswert aufweisen, darstellt.

3. Verfahren nach Ansprüchen 1 oder 2, bei dem die Dosierungspunkte einem oder mehreren der folgenden Punkte entsprechen:
i) Planung von Dosierungspunkten in einem Therapieplan, bei denen die Dosierungspunkte und Voxel verschiedene Erst-und Zweitauflösungen aufweisen, so dass mindestens ein Teil der Dosierungspunkte bezüglich der Voxel im volumetrischen Datensatz verschoben ist, wobei die Transformation die Plan-Dosierungspunkte und die Voxel ausrichtet; oder
ii) Übermittlung von Dosierungspunkten von einem Radiotherapie-Zuführsystem bei denen die Dosierungspunkte und Voxel verschiedene Erst-und Zweitauflösungen aufweisen. Die Transformation führt die Ausrichtung der übermittelten Dosierungspunkte und Voxel durch.

4. Verfahren nach allen vorherigen Ansprüchen bei denen die Transformation mindestens einen der folgenden Punkte beinhaltet: i) Änderung einer Zieldosierungsauflösung der Dosierungspunkte und ii) Durchführung einer Interpolation zwischen den Dosierungspunkten zur Ausrichtung mit einer Voxel-Bildauflösung des volumetrischen Datensatzes mit den Dosierungspunkten.

5. Verfahren nach allen vorherigen Ansprüchen, wobei die RT Objekte einen oder mehrere der folgenden Punkte einschließen;
i) ein erster Satz von RT Objekten, der die anatomische Information beinhaltet und ein zweiter Satz von RT Objekten, der Dosierungsdaten einschließt; oder
ii) ein Satz von, durch ein Therapieplanungssystem erstellten, DICOM RT Objekten, bei denen der DICOM RT Satz mindestens einen der folgenden Punkte darstellt: i) das externe System, wobei der Satz von DICOM RT Objekten mit Information über die anatomische Struktur des Behandlungsbereichs in Hinsicht auf das Bildkoordinatensystem des volumetrischen Datensatzes definiert ist oder ii) die Information hinsichtlich der geplanten Dosierungen für eine Vielzahl von Punkten, die auf dem Behandlungsbereich verteilt sind, wobei mindestens ein Teil der Punkte mit den Voxel im volumetrischen Datensatz verschoben ist; oder
iii) ein Satz von, durch ein oder mehrere externe Planungssysteme erstellten, RT Objekten, wobei der Herstellungsvorgang den Zugriff auf eine DICOM Datenbank einschließt, die RT Objekte speichert.

6. Verfahren nach allen vorherigen Ansprüchen, wobei die Wiedergabe die Anzeige der Bildausschnitte aus axialen, koronalen und sagittalen Ansichtsebenen einschließt, mit darin eingeblendeter Dosierungsmarkierung.

7. Verfahren nach allen vorherigen Ansprüchen, bei dem die Rechen-und Anzeigevorgänge durch ein RT-EPR System gestartet werden, das klinische Informationen in Bezug auf den Patientenstatus und den Behandlungsplan des Patienten erfasst.

8. Verfahren nach allen vorherigen Ansprüchen, darüberhinaus:
Berechnung eines volumetrischen Histogramms (DVH = Dose Volumetric Histogram) auf Grundlage des volumetrischen Datensatzes und den Dosierungswerten und
Wiedergabe von Bildausschnitten des volumetrischen Datensatzes mit Hilfe einer Viewer-Workstation mit Dosierungsmarkierungen, die mit in die Bildausschnitte eingeblendeten Dosierungsdaten angezeigt werden, wobei die Dosierungsmarkierung auf DVH Daten beruhen.

9. Ein computergesteuertes Patientenaktensystem (RT-EPR) mit integriertem Bildarchiv-Computersystem (PACS) zur Bereitstellung der Radiotherapie-Workflow-Verwaltung bezüglich eines Therapieplans auf Grundlage standardmäßiger Ausrichtungsorientierungen (axialer, sagittaler, koronaler), wobei das System Folgendes einschließt:
Eine Workstation;
eine Kommunikationsschnittstelle die so konfiguriert ist, dass ein Bildobjekt abgerufen wird, das einen volumetrischen Datensatz von Voxel beinhaltet, der Abstände und Positionen in Bezug auf ein Bild-Koordinatensystem zur Verfügung stellt;
die Kommunikationsschnittstelle die zum Erhalt von RT Objekten konfiguriert ist, die anatomische Information bezüglich einer anatomischen Struktur (RT Struktur) eines Behandlungsbereichs beinhalten und Dosierungsdaten (RT Daten) einschließen, die Dosierungswerte für Dosierungspunkte im Behandlungsbereich bestimmen, wobei die Dosierungspunkte hinsichtlich der Voxel des volumetrischen Datensatzes verschoben sind;
Speicher zur Speicherung von Programmanweisungen,
**gekennzeichnet durch** einen Prozessor, der die Programmanweisungen ausführt um:
eine Transformation zu berechnen, die Dosierungspunkte mit den Voxel im volumetrischen Datensatz ausrichtet;
mindestens eine Isodosenkurve auf Grundlage des volumetrischen Datensatzes und den Dosierungswerten zu berechnen; und
die Workstation zur Anzeige von axialen, koronalen, sagittalen Ansichten von Bildausschnitten des volumetrischen Datensatzes zu führen, unter Einblendung der Dosierungsmarkierungen in den Bildausschnitten. Die Dosierungsmarkierungen zeigen die Dosierungsdaten auf der Grundlage der Isodosenkurve an und beinhalten eine Konturführung (1010) zur Festlegung eines Bereichs, der eine bestimmte Dosierungsmenge erhält.

10. Das System nach Anspruch 9, in dem die Datenverarbeitung weitergehend konfiguriert wird, um eine oder mehrere der folgenden Punkte zu leisten:
i) Darstellung der Dosierungsmarkierungen für Behandlungsbereiche, die höhere Dosierungswerte als ein ausgewählter Dosierungswert aufweisen.
ii) Darstellung der Dosierungspunkte als Plan-Dosierungspunkte in einem Therapieplan, wobei die Plan-Dosierungspunkte und Voxel unterschiedliche Erst-und Zweitauflösungen aufweisen und die Transformation die Plan-Dosierungspunkte und die Voxel ausrichtet; oder
iii) Darstellung der Dosierungspunkte als Übermittlungs-Dosierungspunkte von einem Radiotherapie-Zuführsystem bei denen die Dosierungspunkte und Voxel verschiedene Erst-und Zweitauflösungen aufweisen. Die Transformation führt die Ausrichtung der übermittelten Dosierungspunkte und Voxel durch.

11. Das System nach Anspruch 9 oder 10, bei dem die Transformation die Änderung einer Zieldosierungsauflösung der Dosierungspunkte beinhaltet, zur Ausrichtung mit einer Voxel-Bildauflösung des volumetrischen Datensatzes.

12. Das System nach jedem vorherigen Anspruch von 9 bis 11, wobei ein erster Satz von RT Objekten die anatomische Information beinhaltet und ein zweiter Satz von RT Objekten die Dosierungsdaten einschließt.

13. Das System nach jedem vorherigen Anspruch von 9 bis 12, wobei die RT Objekte einen Satz von DICOM RT Objekten einschließen, die von einem Therapieplanungssystem erstellt werden. Dabei vertreten die DICOM RT Objekte das externe System, da der Satz von DICOM RT Objekten Informationen der anatomischen Struktur des Behandlungsbereiches enthält, der in Bezug zum Bildkoordinatensystem des volumetrischen Datensatzes definiert wird.

14. Das System nach jedem vorherigen Anspruch von 9 bis 13, wobei die RT Objekte einen Satz von DICOM RT Objekten einschließen, die von einem Therapieplanungssystem erstellt wurden. Dabei stellen die DICOM RT Objekte Information hinsichtlich der geplanten Dosierungen für eine Vielzahl von Punkten bereit, die auf dem Behandlungsbereich verteilt sind, wobei mindestens ein Teil der Punkte mit den Voxel im volumetrischen Datensatz verschoben ist.

15. Das System nach jedem vorherigen Anspruch von 9 bis 14, wobei die Wiedergabe die Anzeige der Bildausschnitte aus axialen, koronalen und sagittalen Ansichtsebenen, mit darin eingeblendeter Dosierungsmarkierung, einschließt.

16. Das System nach jedem vorherigen Anspruch von 9 bis 15, das darüberhinaus eine DICOM Datenbank einschließt, die RT Objekte und Bildobjekte speichert.

## Revendications

1. Un procédé de gestion des opérations de radiothérapie lié à un plan de traitement fondé sur des vues d'orientation standards (axiales, sagittales, coronales) sur système électronique de dossiers de patients de radiothérapie (RT-EPR) intégré à un système informatique d'archives photographiques (PACS), le procédé comprenant les étapes suivantes :
Obtention d'un objet image comprenant un ensemble de données volumétriques de voxels possédant un espacement et une position par rapport au système de coordonnées de l'image ;
Obtenir des objets Dicom RT comprenant des informations anatomiques d'une structure anatomique d'une région de traitement (Structure RT) et comprenant des données de dose (Dose RT) définissant les valeurs de dose pour les points de dose dans la région de traitement, les points de dose étant mal alignés avec les voxels de l'ensemble de données volumétriques,
**caractérisé par** les étapes suivantes :
calcul d'une transformation pour aligner les points de dose avec les voxels dans l'ensemble des données volumétriques ;
calcul d'au moins une courbe d'isodose fondée sur l'ensemble des données volumétriques et des valeurs de dose ;
affichage, par l'intermédiaire de la visionneuse d'une station de travail, de la vue axiale, de la vue coronale et de la vue sagittale de tranches d'images à partir de l'ensemble des données volumétriques avec des indices de dose superposés sur les tranches d'images, les indices de dose, étant indicatifs des données de dose et fondés sur la courbe d'isodose, comprenant un contour (1010) désignant une région qui reçoit une quantité sélectionnée de dose.

2. Le procédé selon la revendication 1, qui comprend en outre la présentation des indices de dose pour des régions de traitement ayant des valeurs de dose plus élevées qu'une valeur de dose sélectionnée.

3. Le procédé selon la revendication 1 ou 2, dans laquelle les points de dose représentent un ou plusieurs des éléments suivants :
i) les points de dose d'un plan dans un plan de traitement, les points de dose du plan et les voxels présentant des premières et secondes résolutions différentes, de telle sorte qu'au moins une partie des points de dose soit mal alignée avec les voxels dans l'ensemble des données volumétriques, la transformation alignant les points de dose du plan et les voxels ; ou
ii) les points de dose de distribution d'un système de distribution de radiothérapie, les points de dose de distribution et les voxels présentant des premières et secondes résolutions différentes, la transformation alignant les points de dose de distribution et les voxels.

4. Le procédé selon l'une des revendications précédentes, la transformation comprenant au moins l'une des actions suivants i) changement d'une résolution de dose cible des points de dose, et ii) accomplissement d'une interpolation entre les points de dose pour aligner une résolution de voxels d'image de l'ensemble des données volumétriques avec les points de dose.

5. Le procédé selon l'une des revendications précédentes, où les objets RT comprennent l'un des éléments suivants :
i) un premier ensemble d'objets RT comprenant des informations anatomiques et un second ensemble d'objets RT comprenant les données de dose ; ou
ii) un ensemble d'objets DICOM RT générés par un système de planification de thérapie, l'ensemble des objets DICOM RT représentant au moins l'un des éléments suivants : i) le système externe, l'ensemble des objets DICOM RT comprenant des informations sur la structure anatomique de la région de traitement définie par rapport au système des coordonnées d'image de l'ensemble des données volumétriques, ou ii) des informations sur les doses prévues pour une pluralité de points distribués dans toute la région de traitement, où au moins une partie des points est mal alignée avec les voxels dans l'ensemble des données volumétriques ; ou
iii) un ensemble d'objets RT générés par un ou plusieurs systèmes de planification externes, l'opération d'obtention comprenant l'accès à une base de données DICOM qui stocke les objets RT.

6. Le procédé selon l'une des revendications précédentes, où l'affichage comprend l'affichage des tranches d'images le long de plans de vues axiales, coronales et sagittales, avec les indices de dose figurant dessus.

7. Le procédé selon l'une des revendications précédentes, où les opérations de calcul et d'affichage sont lancées par un système RT-EPR qui collecte des informations cliniques sur l'état d'un patient et sur le plan de soins d'un patient.

8. Le procédé selon l'une des revendications précédentes :
calcul des données volumétriques de dose d'un histogramme (DVH) sur la base de l'ensemble de données volumétriques et des valeurs de dose et
affichage, par l'intermédiaire de la visionneuse de la station de travail, des tranches d'images à partir de l'ensemble de données volumétriques avec des indices de dose indicatifs des données de dose superposées sur les tranches d'images, les indices de dose étant fondés sur des données DVH

9. Un registre électronique des dossiers des patients de radiothérapie (RT-EPR) intégré à un système informatique d'archives photographiques (PACS) pour permettre la gestion des opérations de radiothérapie liée à un plan de traitement fondé sur des vues d'orientation standards (axiales, sagittales, coronales), le système comprenant :
une station de travail ;
une interface de communication configurée pour obtenir un objet image comprenant un ensemble de données volumétriques de voxels ayant un espacement et une position par rapport au système de coordonnées de l'image ;
l'interface de communication configurée pour obtenir des objets RT comprenant des informations anatomiques d'une structure anatomique (Structure RT) d'une région de traitement et des données de dose (Dose RT) définissant les valeurs de dose pour les points de dose dans la région de traitement, les points de dose étant mal alignés avec les voxels de l'ensemble des données volumétriques :
des instructions d'un programme de stockage de mémoire,
**caractérisées en ce qu'**elles comprennent un processeur configuré pour exécuter les instructions du programme pour :
calculer une transformation pour aligner les points de dose avec les voxels dans l'ensemble de données volumétriques ;
calculer au moins une courbe d'isodose fondée sur l'ensemble des données volumétriques et des valeurs de dose ; et
ordonne à la station de travail d'afficher la vue axiale, la vue coronale et la vue sagittale de tranches d'images à partir de l'ensemble des données volumétriques avec des indices de dose superposés sur les tranches d'images, les indices de dose, indicatifs des données de dose et fondés sur la courbe d'isodose, comprenant un contour (1010) désignant une région qui reçoit une quantité sélectionnée de dose.

10. Le système de la revendication 9, dans laquelle le traitement est en outre configuré pour exécuter une ou plusieurs des actions suivantes :
i) présenter des indices de dose pour des régions de traitement présentant des valeurs de dose plus élevées qu'une valeur de dose sélectionnée ;
ii) représenter les points de dose comme points de dose d'un plan dans un plan de traitement, les points de dose du plan et les voxels présentant des premières et secondes résolutions différentes, la transformation alignant les points de dose de distribution et les voxels ; ou
iii) représenter les points de dose comme points de dose de distribution d'un système de distribution de radiothérapie, les points de dose de distribution et les voxels présentant des premières et secondes résolutions différentes, la transformation alignant les points de dose de distribution et les voxels.

11. Le système selon les revendications 9 ou 10, où la transformation comprend le changement d'une résolution de dose cible des points de dose pour l'aligner avec une résolution de voxels d'image de l'ensemble des données volumétriques.

12. Le système selon l'une des revendications précédentes de 9 à 11, un premier ensemble d'objets RT comprenant les informations anatomiques et un second ensemble d'objets RT comprenant les données de dose.

13. Le système selon l'une des revendications précédentes de 9 à 12, les objets RT comprenant un ensemble d'objets DICOM RT générés par un système de planification de thérapie, l'ensemble des objets DICOM RT représentant le système externe, l'ensemble des objets DICOM RT comprenant des informations sur la structure anatomique de la région de traitement définie par rapport au système de coordonnées d'images de l'ensemble des données volumétriques.

14. Le système selon l'une des revendications précédentes de 9 à 13, où les objets RT comprennent un ensemble d'objets DICOM RT générés par un système de planification de thérapie, l'ensemble des objets DICOM RT représentant des informations sur les doses prévues pour une pluralité de points distribués dans toute la région de traitement, où au moins une partie des points est mal alignée avec les voxels dans l'ensemble des données volumétriques.

15. Le système selon l'une des revendications précédentes de 9 à 14, où l'affichage comprend l'affichage des tranches d'images le long de plans de vues axiales, coronales et sagittales, avec les indices de dose figurant dessus.

16. Le système selon l'une des revendications précédentes de 9 à 15, comprenant en outre une base de données DICOM qui stocke les objets RT et les objets images
